(19) Europäisches Patentamt
European Patent Office
Office européen des brevets

(11) **EP 3 480 208 B1**

(12) **EUROPEAN PATENT SPECIFICATION**

(45) Date of publication and mention of the grant of the patent:
**04.09.2024 Bulletin 2024/36**

(21) Application number: **17819379.3**

(22) Date of filing: **01.07.2017**

(51) International Patent Classification (IPC):
***C07K 7/08*** *(2006.01)* ***A61K 9/107*** *(2006.01)*
***A61K 38/10*** *(2006.01)* ***A61K 39/39*** *(2006.01)*
***A61K 48/00*** *(2006.01)* ***A61P 31/04*** *(2006.01)*
***A61P 31/10*** *(2006.01)*

(52) Cooperative Patent Classification (CPC):
**A61K 39/39; A61K 39/001135; A61K 39/001188; A61P 31/04; A61P 31/10; C07K 7/08;**
A01K 2227/105; A01K 2267/0337; A61K 9/107; A61K 38/00; A61K 2039/53; A61K 2039/55561

(86) International application number:
**PCT/CN2017/091390**

(87) International publication number:
**WO 2018/001383 (04.01.2018 Gazette 2018/01)**

# (54) ANTI-MICROBIAL PEPTIDE DERIVATIVE AND APPLICATION THEREOF

ANTIMIKROBIELLES PEPTIDDERIVAT UND ANWENDUNG DAVON

DÉRIVÉ DE PEPTIDE ANTIMICROBIEN ET SON APPLICATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.07.2016 CN 201610517372**
**01.07.2016 CN 201610517446**

(43) Date of publication of application:
**08.05.2019 Bulletin 2019/19**

(73) Proprietor: **Sichuan University**
**Chengdu, Sichuan 610065 (CN)**

(72) Inventors:
- **YANG, Li**
  **Chengdu**
  **Sichuan 610065 (CN)**
- **MEN, Ke**
  **Chengdu**
  **Sichuan 610065 (CN)**
- **ZHANG, Xueyan**
  **Chengdu**
  **Sichuan 610065 (CN)**
- **HE, Gu**
  **Chengdu**
  **Sichuan 610065 (CN)**
- **WEI, Yuquan**
  **Chengdu, Sichuan 610065 (CN)**

(74) Representative: **Gulde & Partner**
**Patent- und Rechtsanwaltskanzlei mbB**
**Wallstraße 58/59**
**10179 Berlin (DE)**

(56) References cited:
**WO-A1-2006/050611** **WO-A1-2008/022444**
**WO-A1-2014/053624** **WO-A1-99/58141**
**WO-A2-2005/068492** **WO-A2-2007/069090**
**CN-A- 1 901 933** **CN-A- 102 180 951**
**CN-A- 103 467 579** **CN-A- 104 036 155**

- **X. WU ET AL: "In Vitro and In Vivo Activities of Antimicrobial Peptides Developed Using an Amino Acid-Based Activity Prediction Method", ANTIMICROBIAL AGENTS AND CHEMOTHERAPY, vol. 58, no. 9, 1 September 2014 (2014-09-01), US, pages 5342 - 5349, XP055425085, ISSN: 0066-4804, DOI: 10.1128/AAC.02823-14**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- YU JING-MOU ET AL: "Self-aggregated nanoparticles of cholesterol-modified glycol chitosan conjugate: Preparation, characterization, and preliminary assessment as a new drug delivery carrier", EUROPEAN POLYMER JOURNAL, PERGAMON PRESS LTD. OXFORD, GB, vol. 44, no. 3, 16 January 2008 (2008-01-16), pages 555 - 565, XP029230006, ISSN: 0014-3057, DOI: 10.1016/J.EURPOLYMJ.2008.01.013
- BLUHM MARTINA E. C. ET AL: "N-Terminal Ile-Orn- and Trp-Orn-Motif Repeats Enhance Membrane Interaction and Increase the Antimicrobial Activity of Apidaecins against Pseudomonas aeruginosa", FRONTIERS IN CELL AND DEVELOPMENTAL BIOLOGY, vol. 4, 10 May 2016 (2016-05-10), CH, XP055895006, ISSN: 2296-634X, DOI: 10.3389/fcell.2016.00039
- LIU LIHONG ET AL: "Self-assembled cationic peptide nanoparticles as an efficient antimicrobial agent", NATURE NANOTECHNOLOGY, NATURE PUBLISHING GROUP, GB, vol. 4, no. 7, 1 July 2009 (2009-07-01), pages 457 - 463, XP002608981, ISSN: 1748-3395, [retrieved on 20090628], DOI: 10.1038/NNANO.2009.153
- TIAN XIBO ET AL: "Role of peptide self-assembly in antimicrobial peptides : ROLE OF PEPTIDE SELF-ASSEMBLY IN ANTIMICROBIAL PEPTIDES", JOURNAL OF PEPTIDE SIENCE, vol. 21, no. 7, 22 June 2015 (2015-06-22), pages 530 - 539, XP055895372, ISSN: 1075-2617, DOI: 10.1002/psc.2788
- LIU, X.W. ET AL.: "Novel Antimicrobial Peptide-Modified Azithromycin-Loaded Liposomes Against Methicillin-Resistant Staphylococcus Aureus", INTERNATIONAL JOURNAL OF NANOMEDICINE, vol. 11, 14 December 2016 (2016-12-14), pages 6781 - 6794, XP055560052
- MCPHEE, J. B . ET AL.: "Design of Host Defence Peptides for Antimicrobial and Immunity Enhancing Activities", COMBINATORIAL CHEMISTRY & HIGH THROUGHPUT SCREENING, vol. 8, 31 December 2005 (2005-12-31), pages 257 - 272, XP009051307

## Description

### Technical Field

The invention relates to the field of biological medicine and mainly to an antimicrobial peptide derivative and a use thereof, in particular to a hydrophobically modified antimicrobial peptide DP7 derivative and a use thereof.

### Background of the Invention

Antimicrobial peptides, also known as host defense peptides (HDPs), are generally composed of 12 to 100 amino acid residues and are basic polypeptides, most of which are positively charged. Due to their broad-spectrum antibacterial effect, the antimicrobial peptides can effectively inhibit and kill pathogens such as fungi, viruses and parasites, and can selectively kill tumor cells. Moreover, the antimicrobial peptides are not easy to develop drug resistance, thus they constitute the first barrier for host defense against invasion of pathogenic microorganisms and are an important component of the body's immune system. The antimicrobial peptides have become a potential drug for the prevention and control of disease and have a broad development prospect as a substitute for antibiotics. Despite great application potential, only a few antimicrobial peptides are widely used clinically, mainly because of their stability and toxicity. Some researches suggest that the antimicrobial peptides can obtain better stability and reduce toxicity through nanocrystallization, which provides a new research direction for the application of antimicrobial peptides.

The immunomodulatory activities of antimicrobial peptides or HDPs have attracted more and more attention, including inducing the production of cytokines and chemokines by changing signal pathways, directly or indirectly recruiting effector cells including phagocytes, enhancing intracellular and extracellular bacterial killing, promoting dendritic cell maturation and macrophage differentiation, and mediating wound repair and apoptosis. Many antimicrobial peptides exhibit adjuvant activity due to their good immunomodulatory effects, which are achieved mainly by activating the natural immune response and mediating the acquired immune response.

The antimicrobial peptide DP7 is a type of antimicrobial peptide with higher bacterial recognition specificity and stronger antibacterial activity, which is obtained by replacing two amino acids of the template antimicrobial peptide HH2 (Patent No.: WO08022444) according to the computer aided design-based new antimicrobial peptide screening method. Researches show that the antimicrobial peptide DP7 has better antibacterial activity, lower hemolytic toxicity of red blood cells and stronger immunomodulatory activity than HH2. In the study of antibacterial activity in vitro, the antimicrobial peptide DP7 can obviously destruct bacterial cell wall and disrupt cell membrane, thus realizing antibacterial function. In the study of antibacterial activity in vivo, it is found from the abdominal cavity model of mice infected with pathogenic staphylococcus aureus that DP7 has a very good therapeutic effect by inducing immune cells to remove bacteria. However, the high concentration of DP7 will cause hemolysis of red blood cells and kill mice after administration by intravenous injection, indicating that the high concentration of DP7 is toxic to red blood cells and will destroy red blood cells. DP7 is a positively charged hydrophilic antimicrobial peptide and can be modified with a hydrophobic fragment to obtain an amphiphilic compound which can be self-assembled into nanoparticles, so as to greatly reduce the toxicity of intravenous administration, maintain its antibacterial and immunomodulatory effects in vivo, and act as a delivery system for small nucleic acid drugs. Thus, DP7 has a wide use in the drug field.

In Wu et al, "In vitro and in vivo activities of antimicrobial peptides developed using an amino acid-based activity prediction method" ANTIMICROBIAL AGENTS AND CHEMOTHERAPY, 2014, vol. 58, pages 5342-5349, DP7 is disclosed as a positively charged, antimicrobial peptide (VQWRIRVAVIRK).

Examples of N-terminally modified, positively charged antimicrobial peptides of the prior art are disclosed in:

CN 103 467 579 B;
Bluhm et al, "N-terminal Ile-Om- and Trp-Om-motif repeats enhance membrane interaction and increase the antimicrobial activity of Apidaecins against Pseudomonas aeruginosa", FRONTIERS IN CELL AND DEVELOPMENTAL BIOLOGY, 2016, vol. 4;
Liu Lihong et al, "Self-assembled cationic peptide nanoparticles as an efficient antimicrobial agent", NATURE NANOTECHNOLOGY, 2009, vol. 4, pages 457-463;
Tian Xibo et al, "Role of peptide self-assembly in antimicrobial peptides", JOURNAL OF PEPTIDE SCIENCE, 2015, vol. 21, pages 530-539.

### Summary of the Invention

The present invention is directed to subject matter as defined in the claims.

The purpose of the invention is to provide a new modified antimicrobial peptide, a new and effective choice for anti-infection treatment, preparation of a new immunologic adjuvant, and preparation of siRNA carrier in the field.

To solve the above technical problems, the technical scheme adopted by the invention is to provide a hydrophobically modified antimicrobial peptide, and the hydrophobic modification is to couple a hydrophobic fragment at the nitrogen terminal of the antimicrobial peptide (or couple a hydrophobic compound at nitrogen terminal). The amino acid sequence of the antimicrobial peptide DP7 is VQWRIRVAVIRK (SEQ ID No.1).C-terminal of DP7 is usually modified by amidation to improve its stability, when the structure is VQWRIRVAVIRK-$NH_2$.

For the hydrophobically modified antimicrobial peptide, the hydrophobic compound (hydrophobic fragment) is succinylated cholesterol.

For the hydrophobically modified antimicrobial peptide, the nitrogen terminal of the antimicrobial peptide is coupled to the hydrophobic segment (hydrophobic compound) by the amidation reaction of -CO-OH on the hydrophobic segment (hydrophobic compound) with -$NH_2$ on the antimicrobial peptide.

For the hydrophobically modified antimicrobial peptide, the structure is:

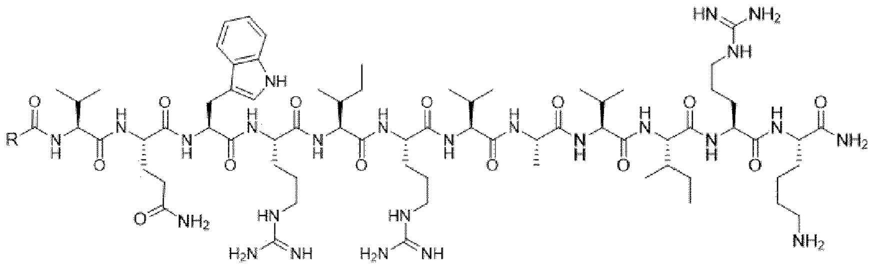

Wherein the R is a cholesterol compound, cholic acid or long-chain fatty acid.

For the hydrophobically modified antimicrobial peptide, the R is

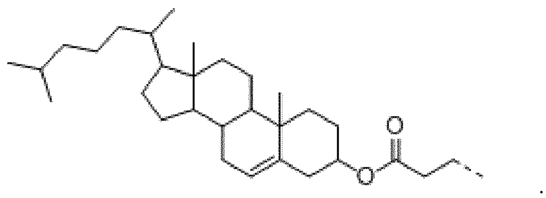

The invention further provides a micelle prepared from the hydrophobically modified antimicrobial peptide.

The micelle is self-assembled from the hydrophobically modified antimicrobial peptide in solution.

The micelle is subjected to freeze-drying treatment.

The micelle is further loaded with at least one of nucleic acid, small molecule drug, polypeptide or protein.

The invention further provides a method for prepare the hydrophobically modified antimicrobial peptide, comprising the following steps:

a Providing the polypeptide VQWRIRVAVIRK-$NH_2$;
b Coupling the hydrophobical fragment at nitrogen terminal of the polypeptide VQWRIRVAVIRK-$NH_2$.

The invention further provides a use of the hydrophobically modified antimicrobial peptide or the micelle in preparing an antimicrobial drug.

For the use in preparing the antimicrobial drug, the antimicrobial is antibacterial or antifungal.

For the use in preparing the antimicrobial drug, the bacteria are at least one of Staphylococcus aureus, Escherichia colibacillus, Acinetobacter baumanmii, Pseudomonas aeruginosa or Salmonella typhi.

For the use in preparing the antimicrobial drug, the fungi are at least one of Canidia Albicans or Candida parapsilosis.

The invention provides an antimicrobial drug prepared from the hydrophobically modified antimicrobial peptide or the micelle as the main active ingredient.

Further, the antimicrobial drug comprises other antimicrobial drugs.

Further, the other antimicrobial drugs are antibiotics.

Wherein, for the antimicrobial drug, the antibiotics are at least one of glycopeptide antibiotic, aminoglycoside antibiotic, macrolide antibiotic and β-lactam antibiotic.

Wherein, for the antimicrobial drug, the β-lactam antibiotic is at least one of penicillin antibiotic or cephalosporin antibiotic.

Further, the penicillin antibiotic is at least one of penicillin G, penicillin V, flucloxacillin, oxacillin, ampicillin, carboxybenzylpenicillin, pivampicillin, sulbenicillin, ticarcillin, piperacillin or amoxicillin. The cephalosporin antibiotic is at least one of cefadroxil, cefalexin, cefazolin, cefradine, cefprozil, cefuroxime, cefaclor, cefamandole, cefotaxime, ceftriaxone, cefixime, cefdinir, cefpirome, cefepime or cefuzonam.

Wherein, for the antimicrobial drug, the aminoglycoside antibiotic is at least one of streptomycin, gentamicin, kanamycin, tobramycin, amikacin, neomycin, sisomicin, tobramycin, amikacin, netilmicin, ribozyme, micronomicin or azithromycin.

Wherein, for the antimicrobial drug, the polypeptide antibiotic is at least one of vancomycin, norvancomycin, polymyxin B or teicoplanin.

Wherein, for the antimicrobial drug, the macrolide antibiotic is at least one of erythromycin, albomycin, odorless erythromycin, erythromycin estolate, acetylspiramycin, midecamycin, j osamycin or azithromycin.

Further, the dosage form of the antimicrobial drug is injection.

The invention further provides a use of the hydrophobically modified antimicrobial peptide or the micelle in preparing an immunologic adjuvant.

The invention further provides an immunologic adjuvant prepared from the hydrophobically modified antimicrobial peptide or the micelle as the immunologic adjuvant and antigen.

The immunologic adjuvant further comprises a single-stranded oligodeoxyribonucleotide (CpG ODNs). Further, the ratio of the hydrophobically modified antimicrobial peptide to CpG ODNs is 1:0.5 to 1:5.

The invention further provides a use of the hydrophobically modified antimicrobial peptide or the micelle in preparing a nucleic acid transporter.

For the use in preparing the nucleic acid transporter, the nucleic acid is RNA.

For the use in preparing the nucleic acid transporter, the nucleic acid is of message RNA, siRNA (small interfering RNA) for RNA interference, or SG RNA (small guide RNA) for genome editing.

The invention further provides a nucleic acid transporter obtained by the nucleic acid loaded in the hydrophobically modified antimicrobial peptide or the micelle.

For the nucleic acid transporter, the nucleic acid is RNA.

For the nucleic acid transporter, the nucleic acid is message RNA (mRNA), siRNA (small interfering RNA) for RNA interference, or sgRNA (small guide RNA) for genome editing.

For the nucleic acid transporter, the ratio by mass of the hydrophobically modified antimicrobial peptide to the nucleic acid is 1: 1 to 20:1.

The invention further provides a method for preparing the nucleic acid transporter, comprising the following steps:

a weighing a proper amount of the hydrophobically modified antimicrobial peptide according to any one of claims 1-9, adding a solution to dissolve, and spontaneously forming micelles; or taking the micelle according to any one of claims 9 - 12 to form a solution;

b adding the nucleic acid into the micellar solution, and incubating at room temperature.

The coupled product of the antimicrobial peptide DP7 and the hydrophobic fragment can be stored in the form of lyophilized powder, and can be directly dissolved in sterile water or physiological saline in use.

The beneficial effects of the invention are as follows: due to small molecular weight, the antimicrobial peptide DP7 in the hydrophobically modified antimicrobial peptide DP7 can be conveniently synthesized by an Fmoc solid phase polypeptide synthesis method, and coupled to a hydrophobic segment by a chemical synthesis coupling method in a simple and easy way. The hydrophobically modified antimicrobial peptide DP7 of the invention can be self-assembled into micelles, develop better monodispersity and Zeta potential, and maintain stable after freeze-drying and redissolution. The micelles of the hydrophobically modified antimicrobial peptide DP7 can significantly reduce the toxicity of the antimicrobial peptide DP7 on red blood cell lysis and realize intravenous administration. Despite the extremely low antibacterial activity in vitro, the hydrophobically modified antimicrobial peptide DP7 has good antibacterial activity in zebrafish and mice. The antibacterial activity in vivo is achieved not by direct sterilization, but by recruiting macrophages, monocytes, neutrophils and other lymphocytes and regulating the expression of some immune cytokines, so as to provide the immune protection for the organism. In the meantime, the hydrophobically modified antimicrobial peptide DP7 can also be used as an immunologic adjuvant to induce a high immune response against the target antigen. In addition, the hydrophobically modified antimicrobial peptide DP7 cationic micelle of the invention can efficiently compound siRNA and introduce it into tumor cells such as colon cancer cells and melanoma cells, inhibit tumor tissue growth by intraperitoneal injection, intratumoral injection and caudal vein injection, and present high safety.

The English abbreviations involved in the invention are as follows:

(1) Vancomycin (VAN);

The strains involved in the invention are as follows:

(1) Staphylococcus aureus: ATCC 33591, ATCC 25923;
(2) Escherichia colibacillus: ATCC 25922;
(3) Pseudomonas aeruginosa: ATCC 10145, ATCC 10145GFP

## Brief Description of the Drawings

Fig. 1 is a route map for synthesizing the antimicrobial peptide DP7 and a hydrophobic fragment conjugate.
Fig. 2 is a mass spectrum of the modified DP7 hydrophobic fragment, wherein A is a mass spectrum of the cholesterol modified DP7-C, and B is a mass spectrum of stearic acid modified DP7-SA.
Fig. 3 is self-assembly of DP7-C micelles and determination of critical micelle concentration, wherein A is a schematic diagram of self-assembly of DP7-C micelles, and B is the determination of the critical micelle concentration of DP7-C micelles.
Fig. 4 is the physical characteristics of DP7-C micelles, wherein A is particle size; B is Zeta potential; C is an atomic force micrograph; and D is the appearance.
Fig. 5 is the comparison of hemolytic activity of DP7 and DP7-C micelles in vitro, wherein A is a curve graph of hemoglobin determination; B is the appearance of hemolysis test.
Fig. 6 indicates the antibacterial activity of DP7-C in vivo, wherein A is an overall and fluorescence diagram of the abdominal cavity of zebrafish infected with Pseudomonas aeruginosa (model); B is a schematic diagram of fluorescence gray integral; and C is a schematic diagram of colony count of the abdominal cavity of mice infected with Staphylococcus aureus (model).
Fig. 7 indicates the study on antibacterial mechanism in vivo of DP7-C, wherein A is a test flow pattern of mononuclear cells and neutrophils in abdominal cavity after DP7-C stimulation; B is a test flow pattern of macrophage in abdominal cavity after DP7-C stimulation; C is a schematic diagram comparing the number of total cells, macrophages, neutrophils and monocytes in the abdominal cavity after DP7-C stimulation; D is a comparison of the expression of immune-related cytokines tested by Q-PCR after DP7-C stimulates PBMC; and E is a comparison of the expression of immune-related cytokines tested by Q-PCR after DP7-C is combined with LPS to stimulate PBMC.
Fig. 8 indicates the test of immune effect of DP7-C/CpG complex; wherein A is a schematic diagram of anti-OVA antibody titers of different immune groups on week 5; B is a growth curve of tumor in each group of mice in the preventive model; C is the change of survival time of mice in preventive model; and D is a growth curve of tumor in each group of mice in the therapeutic model.
Fig. 9 is an experiment of promoting the maturation of dendritic cells by DP7-C.
Fig. 10 is an experiment of promoting DC uptake of OVA antigen by DP7-C.
Fig. 11 is an experiment of stimulating innate immune response by CpG/DP7-C complex.
Fig. 12 is an experiment of stimulating cellular immune response by CpG/DP7-C complex.
Fig. 13 is an experiment of stimulating high-efficiency anti-tumor immunity by CpG/DP7-C adjuvant.
Fig. 14 is an electron microscopic morphology of DP7-C micelle/siRNA complex, wherein A is DP7-C micelle; and B is DP7-C/siRNA complex.
Fig. 15 indicates the test of transfection efficiency in vitro of DP7-C carrying siRNA, wherein A is a comparison of DP7-C with PEI25K and lipofectamine2000 carrying siRNA transfecting tumor cells; B is a schematic diagram of transfection efficiency of B16 cells tested by flow cytometry; and C is schematic diagram of transfection efficiency of C26 cells tested by flow cytometry.
Fig. 16 indicates the cytotoxicity assay of DP7-C (CCK-8 method).
Fig. 17 is a C26 mice model of metastatic colon cancer in the abdominal cavity treated by DP7-C- transmitted VEGF siRNA.
Fig. 18 is a C26 mice colon cancer subcutaneously implanted tumor model treated by DP7-C-transmitted VEGF siRNA.
Fig. 19 is a B16 mice model of metastatic melanoma in the lung treated by DP7-C-transmitted VEGF siRNA.

## Detailed Description of the Preferred Embodiment

DP7 is a positively charged hydrophilic antimicrobial peptide and generally modified by C-terminal amidation. DP7 used in the embodiment of the invention is QWRIRVAVIRK-$NH_2$. The hydrophobic fragments (or hydrophobic compounds) such as cholesterol, cholic acid and long-chain fatty acids may be coupled to the hydrophilic polypeptides, and may be self-assembled into nanostructures. In this study, the antimicrobial peptide DP7 was nanocrystallized by its

coupling with the hydrophobic fragment, thus increasing its stability and reducing hemolytic toxicity, and realizing its intravenous administration. Because of its direct antibacterial and immunomodulatory effects, the nanoparticles formed by the hydrophobically modified DP7 have anti-infection effects.

The antimicrobial peptide DP7 has good immunomodulatory activity and can mediate the natural immune response, which makes it an immunologic adjuvant for vaccines. Co-immunization with antigens can enhance the host's cellular immunity and mediate the production of antigen-specific immunoglobulins. The research results show that the hydrophobic antimicrobial peptides exhibit good adjuvant properties. During in vitro tests, the hydrophobically modified DP7 can be self-assembled into nanoparticles, act as a new immunologic adjuvant by absorbing CpG through electrostatic interaction, enhance the antigen uptake by antigen presenting cells, and promote the maturation of antigen presenting cells and the expression of cytokines. In animal tumor model tests, the hydrophobically modified DP7/CpG complex prolonged the survival time of mice inoculated with tumor, significantly inhibited the growth of tumor and enhanced the level of antigen-specific antibody.

Meanwhile, as the DP7 is positively charged, the hydrophobically modified DP7 micelle formed by self-assembly is also positively charged in aqueous solution, showing its potential as a non-viral gene transmission vector, especially siRNA transmission vector. Combined with siRNA silencing against tumor targets, the hydrophobically modified DP7 will play an important role in the research and application of siRNA-based tumor therapy.

Currently, many studies have been carried out on the formation of nanoparticles by coupling the hydrophobic fragments with water-soluble peptides, and no report has been made on the coupling of the hydrophobic fragments with antimicrobial peptides. The antibacterial and immunomodulatory effects and mechanisms of coupling the hydrophobic fragments with antimicrobial peptides are to be studied. In the study, the cationic micelles formed by DP7-C conjugates successfully realized efficient siRNA introduction to tumor cells, and effectively inhibited the growth of various tumor models to obtain a new siRNA transfer vector. At present, the gene transfer vector based on antimicrobial peptide conjugates has not been reported.

The invention will be described in detail in combination with drawings and embodiment. It should be understood that the examples are only considered to be illustrative for the technical scheme of the invention instead of limitation thereto.

**Example 1 Synthesis of antimicrobial peptide DP7 and cholesterol conjugate (DP7-C)**

The antimicrobial peptide DP7 and the hydrophobic fragment conjugate were synthesized by the synthetic route as shown in Fig. 1, wherein the hydrophobic fragment comprises cholesterol, cholic acid, palmitic acid, stearic acid and lauric acid.

2-chlorotrityl chloride Resin; Fmoc-Rink Amide MBHA Resin, 4-(2', 4'-dimethoxyphenyl-fluorenylmethoxycarbonyl-aminomethyl)-phenoxyl-acetamido-methylbe nzhydrylamine resin; Fmoc, fluorenylmethoxycarbonyl; pbf, tbu, Otbu, Trt and Boc are all protecting groups named 2, 2, 4, 6, 7-pentamethyldihydrobenzofuran-5-sulfonyl, tertiary butyl, tert-butoxy, triphenylmethyl, t-butyloxycarboryl respectively.

The specific synthesis method is as follows:

1 Swelling activation and deprotection of resin:

Swelling: weigh 1.0g of Rink MBHA (4-(2',4'-dimethoxyphenyl-fluorenylmethoxycarbonyl-aminomethyl)-phenoxyl-acetamido-met hylbenzhydrylamine resin) resin (substitution value: 0.36mmol/g) and put into a reaction vessel of a polypeptide synthesizer where the swelling activation is performed by DCM/DMF (1:1); shake the reaction vessel up and down to fully swell the resin, and remove the solvent after about 20min of the swelling process.

Deprotection: remove the Fmoc protecting group from the resin with 15ml of 20% Piperdine/DMF (N,N-dimethylformamide) solution; after 15min of reaction, wash the resin with DCM/DMF (dichloromethane /N, N-dimethylformamide) alternately for three times; wash a small amount of resin with methanol/DCM/DMF in turn, and then add to an EP tube filled with anhydrous ethanol solution containing 5% ninhydrin; bath the tube in boiling water for three minutes until the resin turns into blue, i.e. positive reaction; then wash twice and continue the next reaction, otherwise continue the deprotection step.

2 Coupling of the first amino acid (Lys)

Weigh Fmoc-Lys (Boc)-OH (1.44mmol, 0.93g, 4eq), put into a reaction vessel after dissolved in DMF (about 5ml), add 5ml of HBTu (benzotriazole-N,N,N',N'-tetramethyluronium hexafluophosphate ) (1.44mmol, 0.54g, 4eq) and 5mL of DIEA (diisopropylethylamine) (2.88 mmol, 0.474mL, 8eq) into the DMF solution, and finally supplement 5ml of DMF to start reaction. Shake the reaction vessel up and down for 50min and remove the reaction fluid. Wash a small amount of resin with $CH_3OH$/DCM/DMF in turn, and add to an EP tube filled with absolute ethanol solution containing

5% of ninhydrin. Bath the tube in boiling water for three minutes until the resin turns into yellow or light blue, i.e. negative reaction, which indicates that the reaction is completed. Then, wash the resin in the reaction vessel with DCM/DMF alternately for three times, remove the solvent and carry out the subsequent reaction. If the resin turns into dark blue or reddish brown, the reaction is not complete and the condensation reaction should be repeated. But the condensation reaction is generally complete for it is easy to perform. The raw material used in this step is Fmoc-Rink Amide MBHA Resin comprising Rink Amine Linker modified by MBHA Resin linked to Fmoc. In the invention, the substitution degree is 0.36mmol/g, but other substitution degrees can achieve the same or similar technical effect and are also within the scope of the invention. The substitution degree of 0.36mmol/g used in the invention is the best value obtained by balancing various factors such as yield, purity, resin utilization rate and the like of synthetic fragments.

3 Extension of amino acid chain

After Fmoc is removed from Fmoc-Lys (Boc) -MBHA Resin obtained in step 2, add N,N-dimethylformamide, Fmoc-Arg(pbf)-OH, 1-hydroxybenzotriazole, benzotriazole-N,N,N',N '-tramethyluronium hexafluorophosphate and N,N'-diisopropylethylamine, and allow them to react under nitrogen protection to obtain Fmoc-Arg(pbf)-Lys (Boc) -MBHA Resin. Then connect Fmoc-Ile-OH, Fmoc-Val-OH, Fmoc-Ala-OH, Fmoc-Val-OH, Fmoc-Arg (pbf)-OH, Fmoc-Ile-OH, Fmoc-Arg(pbf)-OH, Fmoc-Trp(Boc)-OH, Fmoc-Gln(Trt)-OH and Fmoc-Val-OH sequentially according to the same method.

The above synthesis yields a fully protected DP7 sequence peptide.

4 Acetylation blocking

In case that the condensation reaction is not ideal, the following steps are generally required so as not to affect the subsequent reaction due to the missing peptide: perform the acetylation blocking on the free amino groups, add the prepared blocking solution (acetic anhydride: pyridine: DMF: 3: 3: 4) to the reaction vessel filled with resin, shake the reaction vessel up and down for about 20min, and test with ninhydrin. If the resin turns into yellow, the reaction is complete and the subsequent reaction could be carried out. In case of incomplete blocking, the blocking time should be prolonged or the ratio of blocking solution should be adjusted to make the reaction as complete as possible.

5 Hydrophobic modification of DP7

1) Hydrophobic modification of DP7 with succinylated cholesterol

Weigh 0.67g (1.44mmol, 4.0 eq) of succinylated cholesterol, dissolve in DCM (about 10ml) and add to the reaction vessel. Then, add 5mL of HBTu (1.44mmol, 0.54g, 4eq) and DIEA (2.88 mmol, 0.474mL, 8eq) into the DMF solution respectively. Shake the reaction vessel up and down for 50min and remove the reaction fluid. Wash a small amount of resin with $CH_3OH$/DCM/DMF in turn, and then add to an EP tube filled with absolute ethanol solution containing 5% of ninhydrin. Bath the tube in boiling water for three minutes until the resin turns into yellow or light blue, i.e. positive reaction, indicating that the reaction is complete. Then, wash the resin in the reaction vessel with DCM/DMF alternately for three times, and remove the solvent. Add the obtained resin into a reaction vessel filled with lysate, seal the reaction vessel, and fix to a cradle for reaction. After about 2h of the reaction process, remove the resin by filtration, wash the resin with DCM for several times, collect the filtrate, remove the TFA and solvent by rotary evaporation, add anhydrous ether of ice to the remaining liquid to produce a large amount of white flocculent precipitate, and centrifuge the white precipitate at high speed (4000r/min) to obtain the crude product. The crude product is further purified and refined by HPLC to obtain the target product, i.e. cholesterol-containing DP7 (DP7-C). Its mass spectrum is shown in Fig. 2A, and the measured molecular weight is as expected.

2) Hydrophobic modification of DP7 with cholic acid

Weigh 0.59g (1.44 mmol, 4.0 eq) of cholic acid, dissolve in DCM (about 10ml), put into a reaction vessel, then add 5ml of HBTU (1.44 mmol, 0.54g, 4 eq) and DIEA (2.88 mmol, 0.474 ml, 8 eq) into the DMF solution respectively. Shake the reaction vessel up and down for 50min and remove the reaction fluid. Wash a small amount of resin with $CH_3OH$/DCM/DMF in turn, and then add to an EP tube filled with absolute ethanol solution containing 5% of ninhydrin. Bath the tube in boiling water for three minutes until the resin turns into yellow or light blue, i.e. negative reaction, indicating that the reaction is complete. Then, wash the resin in the reaction vessel with DCM/DMF alternately for three times, and remove the solvent. Add the obtained resin into a reaction vessel filled with lysate, seal the reaction vessel, and fix to a cradle for reaction. After about 2h of the reaction process, remove the resin by filtration, wash the resin with DCM for several times, collect the filtrate, remove the TFA and solvent by rotary evaporation, add anhydrous ether of

ice to the remaining liquid to produce a large amount of white flocculent precipitate, and centrifuge the white precipitate at high speed (4000 r/min) to obtain the crude product. The crude product is further purified and refined by HPLC to obtain the target product, i.e. cholesterol DP7 (DP7-CA).

3) Hydrophobic modification of DP7 with stearic acid

Weigh 0.41g (1.44 mmol, 4.0 eq) of stearic acid, dissolve in DCM (about 10ml), put into a reaction vessel, then add 5ml of HBTU (1.44 mmol, 0.54g, 4 eq) and DIEA (2.88 mmol, 0.474 ml, 8 eq) into the DMF solution respectively. Shake the reaction vessel up and down for 50min and remove the reaction fluid. Wash a small amount of resin with $CH_3OH$/DCM/DMF in turn, and then add to an EP tube filled with absolute ethanol solution containing 5% of ninhydrin. Bath the tube in boiling water for three minutes until the resin turns into yellow or light blue, i.e. negative reaction, indicating that the reaction is complete. Then, wash the resin in the reaction vessel with DCM/DMF alternately for three times, and remove the solvent. Add the obtained resin into a reaction vessel filled with lysate, seal the reaction vessel, and fix to a cradle for reaction. After about 2h of the reaction process, remove the resin by filtration, wash the resin with DCM for several times, collect the filtrate, remove the TFA and solvent by rotary evaporation, add anhydrous ether of ice to the remaining liquid to produce a large amount of white flocculent precipitate, and centrifuge the white precipitate at high speed (4000r/min) to obtain the crude product. The crude product is further purified and refined by HPLC to obtain the target product, i.e. stearic acid DP7 (DP7-SA). Its mass spectrum is shown in Fig. 2B, and the measured molecular weight is as expected.

4) Hydrophobic modification of DP7 with palmitic acid

Weigh 0.37g (1.44 mmol, 4.0 eq) of palmitic acid, dissolve in DCM (about 10ml), put into a reaction vessel, then add 5ml of HBTU (1.44 mmol, 0.54g, 4 eq) and DIEA (2.88 mmol, 0.474 ml, 8 eq) into the DMF solution respectively. Shake the reaction vessel up and down for 50min and remove the reaction fluid. Wash a small amount of resin with $CH_3OH$/DCM/DMF in turn, and then add to an EP tube filled with absolute ethanol solution containing 5% of ninhydrin. Bath the tube in boiling water for three minutes until the resin turns into yellow or light blue, i.e. negative reaction, indicating that the reaction is complete. Then, wash the resin in the reaction vessel with DCM/DMF alternately for three times, and remove the solvent. Add the obtained resin into a reaction vessel filled with lysate, seal the reaction vessel, and fix to a cradle for reaction. After about 2h of the reaction process, remove the resin by filtration, wash the resin with DCM for several times, collect the filtrate, remove the TFA and solvent by rotary evaporation, add anhydrous ether of ice to the remaining liquid to produce a large amount of white flocculent precipitate, and centrifuge the white precipitate at high speed (4000r/min) to obtain the crude product. The crude product is further purified and refined by HPLC to obtain the target product, i.e. palmitic acid DP7 (DP7-PA).

5) Hydrophobic modification of DP7 with lauric acid

Weigh 0.29g (1.44 mmol, 4.0 eq) of lauric acid, dissolve in DCM (about 10ml), put into a reaction vessel, then add 5ml of HBTU (1.44 mmol, 0.54g, 4 eq) and DIEA (2.88 mmol, 0.474 ml, 8 eq) into the DMF solution respectively. Shake the reaction vessel up and down for 50min and remove the reaction fluid. Wash a small amount of resin with $CH_3OH$/DCM/DMF in turn, and then add to an EP tube filled with absolute ethanol solution containing 5% of ninhydrin. Bath the tube in boiling water for three minutes until the resin turns into yellow or light blue, i.e. negative reaction, indicating that the reaction is complete. Then, wash the resin in the reaction vessel with DCM/DMF alternately for three times, and remove the solvent. Add the obtained resin into a reaction vessel filled with lysate, seal the reaction vessel, and fix to a cradle for reaction. After about 2h of the reaction process, remove the resin by filtration, wash the resin with DCM for several times, collect the filtrate, remove the TFA and solvent by rotary evaporation, add anhydrous ether of ice to the remaining liquid to produce a large amount of white flocculent precipitate, and centrifuge the white precipitate at high speed (4000 r/min) to obtain the crude product. The crude product is further purified and refined by HPLC to obtain the target product, i.e. lauric acid DP7 (DP7-DA).

The solid-phase peptide synthesis technology is almost mature, and the conjugate of the antimicrobial peptide DP7 and the hydrophobic fragment can also be synthesized by relevant companies. For example, the DP7-C modified by succinylated cholesterol (Chol-suc-VQWRIRVAVIRK-$NH_2$) is synthesized by Shanghai Ketai Biotechnology Co., Ltd. based on the solid-phase peptide synthesis method. The synthesized DP7-C is purified by HPLC, with the purity more than 95%; and the molecular weight of DP7-C is determined by MS. The synthesized polypeptide is stored at - 20°C and prepared into 5mg/ml of mother liquor by MillQ water for use. For the DP7-C synthesized by the solid-phase peptide synthesis method (Chol-suc-VQWRIRVAVIRK-$NH_2$), N-terminal is coupled to the hydrophobic cholesterol through ester bonds on the basis of DP7, and C-terminal is connected to a molecule -$NH_2$ for protection. The mass spectrogram of DP7-C is shown in Fig. 2. Its molecular weight is 1991.3408, and the main peak on MS is 996.1748, indicating that the

correct DP7-C is synthesized.

Since the experiments show that the hydrophobically modified DP7 prepared above has similar properties, the following examples are described in detail in combination with cholesterol DP7 (DP7-C).

**Example 2 Critical micelle concentration of DP7-C**

DP7-C can be self-assembled into micelles in aqueous solution. We detected the critical micelle concentration (CMC) of DP7-C by the pyrene fluorescence probe spectroscopy.

Pyrene is insoluble in water, and its solubility in water is about $6\times10^{-7}$mol/L, but it is easily soluble in ethanol and diethyl ether. The fluorescence emission spectrum of pyrene in aqueous solution has five fluorescence peaks, and the ratio of the first emission spectrum light intensity I1 to the third emission spectrum light intensity I3 in aqueous solution (the ratio of fluorescence intensity at 373 nm to that at 384 nm) is about 1.8. According to the literature, the surfactant may solubilize nonpolar organic compounds, and the surfactant at different concentrations may solubilize pyrene to varying degrees. Thus, the solubilizing ability of the solution will have an obvious mutation point after the concentration of surfactant exceeds the critical micelle concentration (CMC). If a curve is drawn according to the change of I1/I3 with the surfactant concentration, the midpoint of the curve mutation is the CMC of the substrate to be tested. Therefore, the CMC of pyrene in the solution can be determined by measuring the fluorescence spectra of pyrene in DP7-C solutions with different concentrations. Detection method of CMC: weigh DP7-C and dissolve with Milliq water in 37°C water bath to obtain DP7-C mother liquor with a concentration of 1.5 mg/mL. Prepare the pyrene solution of $6\times10^{-5}$ mol/L by taking methanol as solvent. After methanol is volatilized and dried in a dark and ventilated place, add 4mL of 1.5mg/mL DP7-C solution, sonicate at 37°C for 4h and shake on a cradle for 8h. Then, detect I1 and I3 by a fluorescence spectrophotometer. Add different volumes of sample mother liquor to BD tubes containing trace pyrene to reach the concentrations of 0.0001, 0.001, 0.01, 0.025, 0.05, 0.10, 0.125, 0.25, 0.5, 1.0 and 1.5mg/ml respectively, and determine the fluorescence spectra of each solution. The critical micelle concentration can be calculated according to the fluorescence spectrum, wherein the excitation wavelength of fluorescence scanning is 334nm, the emission wavelength is 373nm and 384 nm, the excitation slit is set to 8.0nm, the emission slit is set to 2.5nm, and the scanning speed is 1200nm/min.

Fig. 3A is a schematic diagram of the DP7-C self-assembled into micelles in an aqueous solution, of which the CMC measurement value is 3.47μg/mL (see Fig. 3B for the results).

**Example 3 Physical characteristics of DP7-C micelles**

We detected some physical characteristics such as particle size and Zeta potential of DP7-C micelles by the atomic force microscope and Malvin particle size meter.

1 Atomic force microscope photography: prepare different concentrations of DP7-C solution, add to the mica sheets dropwise and dry naturally. Put the mica sheets coated with DP7-C on the atomic force microscope for photography.
2 Detection of particle size and Zeta potential: dissolve DP7-C in Milliq water, determine particle size and Zeta potential by Malvin particle size meter, and detect each sample for four times to obtain their average value.
3 Appearance and solution state of DP7-C: observe the appearance of MilliQ water, DP7-C water solution and lyophilized powder, and take photos with a camera; wherein, a is MilliQ water, b is DP7-C dissolved in aqueous solution, C is the state of DP7-C lyophilized powder after redissolving, and d is the shape and color of DP7-C lyophilized powder.

The results are given in Fig. 4. Specifically, the DP7-C micelle is spherical or ellipsoidal (Fig. 4C), the particle size of DP7-C micelle is about 24.3 ± 1.5 nm (Fig. 4A), and Zeta potential is about 28.8 ± 0.27 mV (Fig. 4B). DP7-C lyophilized powder is white fluffy, soluble in water, and colorless and transparent.

**Example 4 Hemolytic activity of DP7 and DP7-C micelles in vitro**

In the hemolysis test, DP7 and DP7-C at the same concentration were detected for the hemolysis of red blood cells.

1. Take the blood of healthy volunteers and put into an anticoagulant tube and gently mix.
2. Take out the anticoagulant, add isometric physiological saline, mix slowly and evenly, centrifuge at 400× g for 10min, and discard the supernatant.
3. Slowly mix the red blood cells and wash with PBS, centrifuge at 400×g for 10min, discard the supernatant, and wash the cell precipitate with PBS for three times.
4. Dilute with PBS to obtain 20% (v/v) red blood cell solution, which can be used immediately or stored at 4°C for

about 2 weeks (without hemolysis).

5. Dilute the drug into a series of concentration gradients with PBS, add 100μL of drug into a 96-well plate, take 100μL of 2% Twain 20 as the positive control, add 100μL of PBS to the negative control. Repeat the process 3 times for each concentration of drug.

6. Dilute 20% (v/v) of red blood cell solution with PBS according to the volume ratio of 1:20, mix evenly and add to a 96-well plate, with the drug concentration of 100, 200, 400, 600, 800, 1000, 1200, 1600μg/mL respectively, and incubate at 37°C for 1h.

7. Incubate the mixture of drugs and red blood cells, centrifuge at 900×g for 10min, add 160μL of the supernatant to a new 96-well plate, and read the absorbance value at OD 450 nm.

8. According to the absorbance value of A450, calculate the percentage of hemolysis corresponding to each concentration of drugs.

The calculation formula of the percentage of hemolysis is:

$$\frac{\text{Drug treatment group A450-PBS treatment group A450}}{\text{Twain 20 treatment group A450- PBS treatment group A450}} \times 100\%$$

The hemolysis test results are given in Fig. 5A. When the drug concentration is more than 0.8mg/mL, the degree of lysis of DP7-C micelles to red blood cells is much less than DP7, indicating that the antimicrobial peptide DP7 could be coupled to cholesterol for greatly reducing the toxicity to red blood cells. Fig. 5B visually shows the hemolysis of red blood cells under different conditions. Specifically, in the negative control group (PBS) (group a), no hemolysis is observed and red blood cells are settled at the bottom of the vessel. In the positive control group (2% Tween-20) (group b) and DP7 solution group (group c), red blood cells are lysed. In the DP7-C micelles (group d), red blood cells are not lysed but uniformly suspended in the solution due to their micelle characteristics.

**Example 5 Antibacterial activity of DP7-C in vivo**

The previous experiments show that the cholesterol-containing DP7-C micelles have low antibacterial activity in vitro (MIC>1024mg/L for multiple strains). Further, we detected the antibacterial activity of the intra-abdominally infected mouse and zebrafish model in vivo.

(I) Detection of antibacterial activity of DP7-C in mice (Gram-positive bacteria - Staphylococcus aureus)

The anti-infection activity of DP7-C is determined by the intra-abdominally infected models. The experimental groups are as follows:

NS group: 100μL of normal saline per mouse;
DP7-C group: 0.3mg/kg;
VAN positive control group: 10mg/kg.

The experimental steps are as follows:

(1) Activate Staphylococcus aureus 33591 with MHB the day before the experiment, wash the activated strains twice with normal saline on the day of the experiment, and determine the concentration of bacterial liquid for later use.

(2) To establish an abdominally infected mouse model, intraperitoneally inject the bacterial solution of $1.5\times10^8$ cfu/0. 5mL into each mouse.

(3) Intravenously inject the drug by group after one hour of infection, 0. 1mL/mouse.

(4) 24h later, intraperitoneally inject 5mL of normal saline into the mouse, gently massage the abdomen, kill the mouse and disinfect with 75% alcohol. After 5min, scissor the abdominal cavity epithelium, make a small opening in the abdominal cavity, draw ascites as much as possible from the opening with a 1ml syringe, and then transfer to a sterile EP tube for mixing.

(5) Dilute with normal saline at a dilution of, for example, 10×, 100×, 1000× and 10000×; take three bacterial solutions with suitable concentrations, 20μL each, coat the MHA plate and incubate in a bacteria incubator overnight.

(6) Select a plate with 20 - 200 colonies and convert and calculate the amount of bacteria per milliliter in 5 milliliters of ascites.

The therapeutic outcome of DP7-C in treating the abdominally infected mouse model is given in Fig. 6C. The results show that the average colony forming unit (CFU) of Staphylococcus aureus in the DP7-C group (1mg/kg) and the positive drug group (20mg/kg VAN) after intravenous administration are significantly lower than those in NS group ($p<0.01$). In addition, the antibacterial effect of DP7-C group is basically the same as that of the positive drug group, with no statistical difference. It shows that DP7-C has a good antibacterial activity in the systemically infected mouse model.

(II) Antibacterial activity of DP7-C in Zebrafish (Gram-negative bacteria - Pseudomonas aeruginosa)

Next, we established an abdominally infected zebrafish model through Pseudomonas aeruginosa with fluorescence (PAO1-GFP) to further verify the anti-infection effect of DP7-C.

The male and female AB wild zebrafish were mated according to the mating and breeding procedures. After spawning, the roe was collected, placed in an incubator at 28°C and incubated in the seawater containing PTU. The seawater containing PTU was changed once a day. When zebrafish was incubated for 48h, Pseudomonas aeruginosa with fluorescence (PAO1-GFP) diluted with DP7-C and normal saline was intraperitoneally injected, and photographs were taken at 3h, 8h and 18h under a fluorescence microscope to observe the growth of PAO1-GFP in the abdomen of zebrafish.

The results are given in Fig. 6 (A, B). The growth rate of PAO1-GFP in zebrafish abdominal cavity is positively correlated with the total intensity of green fluorescence, and the growth rate of PAO1-GFP of the DP7-C group is much lower than that of NS group, indicating that the DP7-C micelles with low concentration (1mg/mL) have good antibacterial activity in zebrafish.

**Example 6 Study on antibacterial mechanism in vivo of DP7-C**

During the detection of DP7-C antibacterial activity, it was found that DP7-C had low in vitro antibacterial activity but high in vivo antibacterial activity. The possible reason lies in that DP7-C has achieved the antibacterial effect by regulating the organism immune system. We detected the effects of DP7-C on the immune function from the cellular level and cytokine level.

(I) Detection of activated immune-related cells

Grouping: The mice are randomly divided into two groups (NS group and DP7-C group) with 5 mice in each group. The detected immunocytes and their surface markers are shown in the table below:

| | Cell marker |
|---|---|
| Neutrophilic granulocyte | Gr1+(PE), F4/80-(APC) |
| Monocyte | Gr1+(PE), F4/80+(APC) |
| Macrophage | F4/80+(APC), CD11b+(PE) |

Each mouse in the DP7-C group is given 200μL of 1mg/mL DP7-C, while each mouse in the NS group is injected with 200μL of normal saline. The mice of both groups are killed 24h later. Each mouse is intraperitoneally injected with 5mL of normal saline. By gently massaging the abdomen, the ascites in the abdominal cavity is aspirated to calculate the cell concentration and total cell count there. The lymphocyte typing of the ascites is detected by a flow cytometry, the percentage of macrophages, neutrophils and inflammatory monocytes in total cells is analyzed, and the number of macrophages, neutrophils and inflammatory monocytes is calculated according to the results of flow cytometry and the total number of cells in ascites.

The results are given in Fig. 7C. After the mice are intraperitoneally injected with DP7-C, the total number of cells in the abdominal cavity is increased significantly and the number of macrophages, neutrophils and monocytes is significantly different. Especially, the percentage of monocytes is increased from 2.7% to 32.8% (Fig. 7A) and the percentage of macrophages is increased from 38.8% to 50.8% (Fig. 7B).

(II) Determination of immune-related cytokines

In order to determine the changes of cytokines in mouse PBMC after DP7-C stimulation, the isolated mouse PBMC is diluted to $1\times10^6$ cells/mL and added to a 6-well plate at 2ml/well, in which the stimulation concentration of

DP7-C is 200μg/ml and the stimulation time is 4h. After stimulation, the cells are collected and stored in a - 80°C refrigerator. Meanwhile, in order to verify whether DP7-C can reverse LPS-induced inflammatory reaction, an experiment is set up to stimulate PBMC together with LPS: PBMC is diluted to $1\times10^6$ cells/ml and added to a 6-well plate at 2mL/well in which the stimulation concentration of DP7-C is 200μg/mL; after 1h, stimulated by LPS for 4h, and cells are collected and stored in - 80°C refrigerator. After all samples are collected, the following steps are performed, including centrifugation, washing, total RNA extraction, reverse transcription and real-time quantitative PCR.

The expression of cytokines related to PBMC stimulated by DP7-C is shown in Fig. 7D. The expression of IL-1, IL-6, MCP-1, M-CSF, TNF-α and other cytokines related to immune activation is greatly increased; meanwhile, when DP7-C and LPS stimulate PBMC together, it is found that the gene expression of major cytokines related to cytokine storm, such as IL-10, MCP-1 and TNF-α is significantly reduced. This indicates that DP7-C could reduce the degree of injury caused by sepsis and other related infectious diseases.

**Example 7 Study on DP7-C as immunologic adjuvant**

In previous test, it is found that DP7-C could significantly up-regulate the expression of immune-related cytokines in mouse PBMC. Thus, it is predicted that DP7-C combined with CpG ODNs could be used as a new immunologic adjuvant, and DP7-C could spontaneously form micelles, which also inspires the inventors to use it as a possible substitute for aluminum adjuvant. In this study, the inventors studies the immunomodulatory effect of DP7-C/CpG complex adjuvant, and the antitumor effect in the preventive and therapeutic tumor model of the mouse by taking the OVA as the model antigen.

(1) Animal grouping:
C57BL/6J female mice are randomly divided into 4 groups, 10 mice each group as follows:

NS group: 100μL of normal saline;
CpG group: 10μg OVA +20μg CpG
DP7-C group: 10μg OVA + 40μg DP7-C;
CpG/DP7-C group: 10μg OVA + 40μg DP7-C + 20μg CpG

(2) Preventive immunity tumor model: perform subcutaneous immunotherapy at multiple sites on weeks 0, 2 and 4, and detect total antibody titers before tumor vaccination on week 5. Subcutaneously inoculate the tumor cells EG7-OVA: $2\times10^6$ at the back of each mouse. After the tumor grows out, measure at intervals of 3 days. Calculation formula of tumor volume: $0.52 \times \text{length} \times \text{width}^2$.

Therapeutic immune tumor model: subcutaneously inoculate $2\times10^6$ of EG7-OVA tumor cells at the back of each mouse on day 0, and immunize on day 5 (once a week, for 3 consecutive times). Measure at intervals of 3 days and observe the survival time after the tumor grows out.

The results are given in Fig. 8. In the preventive model (A and B), the OVA-specific antibodies produced in the DP7-C/CpG group are significantly higher than those produced in other groups on week 5, showing statistical differences. Tumor growth in DP7-C/CpG group is significantly inhibited on day 26 after tumor inoculation. In the therapeutic model (C and D), the survival time of mice inoculated with tumor is significantly prolonged in the DP7-C/CpG group and the growth of tumor is greatly inhibited. The results show that DP7-C/CpG is a new immunologic adjuvant with good immunostimulatory activity.

**Example 8 Study on the role of DP7-C in promoting antigen uptake in vitro**

The mechanism of adjuvant-activated immune response probably lies in its promotion to the maturation of DC cells to improve their antigen uptake and processing. We studied the effect of DP7-C micelles on DC activity in vitro.

**(I) Promoting the maturation of dendritic cells**

The maturation of dendritic cells (DC) largely determines the immune response or tolerance of the body. Their surface antigens CD80, CD86 and MHC-II are obviously up-regulated with the maturation of DC. Therefore, we detected the effect of DP7-C adjuvant on dendritic cell maturation by flow cytometry.

The experimental steps are as follows:

(1) Isolate C57BL mouse primary bone marrow cells and incubate in the induced medium containing 10ng/ml of GM-CSF and 10ng/ml of IL-4 for 5 days.

(2) Add the DP7-C adjuvant into DC cell culture, mix well, and incubate for 16h.
(3) Collect the stimulated DC cells, wash them twice with PBS, re-suspend the cells in 100μL of PBS, add 1μL of anti-mouse CD16/CD32, and block at 4 °C for 30 min.
(4) Wash with PBS for 3 times, add 1μg of FITC-anti-CD80, APC-anti-CD86 and PE -anti-MHC-II, incubate at room temperature for 30min, wash PBS for 3 times and re-suspend in 200μL of PBS for flow cytometry.

The results are given in Fig. 9. After DP7-C adjuvant is added to DCS cells, the expression of cell maturation molecule MHC II and co-expressed CD80 and CD86 is increased, and their difference from the NS group is statistically significant.

**(II) Enhancing antigen uptake of dendritic cells**

DC cells are the most powerful antigen presenting cells in the body, which can absorb foreign substances, process and present them to T cells to stimulate immune response. DC cells play an important role in T cell immune response and production of T cell dependent antibody. We examined the effect of DP7-C on DC presenting antigen by flow cytometry.
The experimental steps are as follows:

(1) Isolate C57BL mouse primary bone marrow cells and incubate in the induced medium containing 10ng/ml of GM-CSF and 10ng/ml of IL-4 for 5 days.
(2) Add the OVA protein markers FITC, OVA or DP7-C/OVA into DC cell culture, mix well and incubate for 3h.
(3) Collect the stimulated DC cells, wash with PBS twice, re-suspend the cells in 300μL of PBS, and perform flow cytometry.

The results are given in Fig. 10. The uptake rate of OVA antigen by DC cells alone is low, and the uptake of OVA by DC cells is slightly enhanced through 40μg/ml of DP7-C to a certain extent. However, 2.5μg/ml of DP7-C could significantly increase the uptake of antigen by DC cells, and the uptake effect is increased with DP7-C concentration.

**Example 9 DP7-C stimulates innate immune response**

Innate immunity plays an important role in anti-tumor immunity. We detected the killing activity of mouse NK cells after immunization with DP7-C adjuvant.
The experimental steps are as follows:

(1) Preparation of effector cells

After 48h of primary immunization, kill the mice by the cervical dislocation, and isolate splenic lymphocyte from the lymphocyte separation medium as effector cells.

(2) Preparation of target cells

Resuscitate and incubate YAC-1 cells, transfer one day before the experiment, and keep $2\times10^6$/20ml. Stain with trypan blue. Available when activity is more than 95%.

(3) Co-incubation of effector cells and target cells

① Add target cells and effector cells into a 96-well plate (ratio of target cells to effector cells: 1:25, 1:50, 1:100, 1:200 as per 10,000 target cells per well) and incubate at 37°C for 4h.
② Centrifuge at 250g, and take supernatant for LDH releasing test.

The results are given in Fig.11. Specifically, the NS group does not show NK cell activity basically; the CpG group and the DP7-C group show slightly strong NK cell killing activity; and CpG/DP7-C complex could effectively enhance NK cell activity. The differences among them are significant.

**Example 10 DP7-C stimulates cellular immune response**

From the above results, we know that DP7-C could be combined with CpG to stimulate the specific immune response of antigen, effectively inhibit tumor growth and metastasis, and prolong the survival time of mice. Moreover, DP7-C and CpG could significantly up-regulate specific antibody titer of OVA antigen and enhance humoral immune response. So we further detected DP7-C activated cellular immune response.

The specific steps are as follows:

(1) Stimulation of splenic lymphocytes in vitro.

① After three times of immunization in 7 days, separate splenic lymphocytes of mice, count and dilute to $5\times10^6$/ml.
② Put into a 6-well plate as per $5\times10^6$/well, add 10μg/ml of OVA holoprotein to each well, stimulate at 37°C for 1h (negative control: DMSO; positive control: 5 μg/ml conA).
③ Add 1μL of Golgiplug to each well, mix well and incubate for 6-12h.

(2) Fluorescent-labeled antibody

① Collect and add the cells to 2 ml of 1×BD Pharmlyse vortex, incubate for 10min in dark place at room temperature, centrifuge at 500g for 5min, and discard the supernatant.
② After washing with 1×instaining buffer, re-suspend in 100μL of 1×instaining buffer, add 1μL of anti-mouse CD16/CD32, and incubate at 4°C for 15min for blocking.
③ After washing with 1×instaining buffer, re-suspend the cells in 100μl of staining buffer, add 1μl of PE-anti-mouse CD8 and perCP-cy5-anti-mouse CD4 respectively, and incubate at 4°C for 30min in dark place.
④ After washing twice with the staining buffer, add to 250μl fixation/ permeabilization vortex for fully suspend, incubate in dark place at room temperature for 20min, fix and permeabilize the cells.

(7) Add 2mL of BD Perm/wash buffer, centrifuge at 500g for 5min and discard the supernatant.
(8) Re-suspend cells in 100μL of BD perm/wash buffer, add the intracellular antigen antibody (FITC-IFN-γ or PE-IL-17A), and incubate in dark place at room temperature for 30min. Then, add 2ml of BD Perm/wash buffer, centrifuge at 500g for 5min and discard the supernatant. Re-suspend in 500μl of PBS containing 2% paraformaldehyde. When the vortex is fully suspended, the flow cytometry can be performed.

The results are given in Fig. 12. The percentage of $CD4^+$/IFN-$\gamma^+$, $CD8^+$/IFN-$\gamma^+$ and $CD4^+$/IL-$17A^+$ cells is low in the NS group; while the percentage of such cells in mice immunized with CpG and DP7-C adjuvant is increased. However, the percentage of these three cells in mice spleen immunized with CpG/DP7-C complex is high, and the difference is statistically significant compared with the NS group.

**Example 11 CpG/DP7-C adjuvant stimulates high-efficient antitumor immunity**

From the above results, we know that the tumor growth of mice immunized with CpG/DP7-C complex is inhibited in the EG7-OVA tumor model. We further used NY-ESO-1 as antigen to form a vaccine with CpG/DP7-C adjuvant, aim at testing whether the vaccine can inhibit the growth of melanoma with high expression of the antigen.

The specific steps are as follows:

(1) Vaccine preparation

Incubate 20μg of CpG and 40μg of DP7-C at 37°C for 15min, add 5μg of NY-ESO-1 protein, and replenish to a volume of 100μl with sterile PBS.

(2) Grouping and dosage regimen

In this study, animal experiments are divided into 5 groups: NS, CpG, DP7-C and CpG/DP7-C, 10 mice for each group, and the dosage of each mouse is as follows:

① NS group: 100μl of PBS;
② CpG group: 5μg of NY-ESO-1 protein + 20μg of CpG;
③ DP7-C group: 5μg of NY-ESO-1 protein + 40μg of DP7-C;

④ CpG/DP7-C group: 5μg of NY-ESO-1 protein + 20 μg of CpG + 40μg DP7-C;

The total dosage of each mouse is 100μl; if the dosage is less than 100μl, sterile PBS may be added to reach 100μl.

(3) Preventive immunity tumor model: perform subcutaneous immunotherapy at multiple sites on weeks 0, 2 and 4, and detect total antibody titers before inoculate tumor on week 5. Subcutaneously inoculate the tumor cells NY-ESO-$1^+$B16: $2\times10^5$ at the back of each mouse. After the tumor grows out, measure it at intervals of 3 days. Calculation formula of tumor volume: $0.52 \times$ length $\times$width$^2$. Therapeutic immune tumor model: subcutaneously inoculate $2\times10^5$ of NY-ESO-$1^+$B16 tumor cells at the back of each mouse on day 0, and immunize on day 5 (once a week, for 3 consecutive times). Measure at intervals of 3 days and observe the survival time after the tumor grows out.

The results are given in Fig. 13. In both preventive model (Fig. 13A) and therapeutic model (Fig. 13B), the tumors of NS group mice grow rapidly; the CpG alone or DP7-C adjuvant could inhibit tumor growth to some extent; and the immune CpG/DP7-C complex adjuvant could effectively inhibit the growth of tumor, which is significantly different from NS group.

**Example 12 Morphology of DP7-C micelle/siRNA complex under electron microscope**

As the DP7 is positively charged, the DP7-C micelle formed by self-assembly is also positively charged in aqueous solution, showing its potential as a non-viral gene transmission vector, especially siRNA transmission vector. The morphology of DP7-C micelles formed by self-assembly and DP7-C/siRNA complexes is observed under a transmission electron microscopy (H-6009IV, Hitachi, Japan). First, 1mg\mL of micellar solution is diluted with distilled water and coated on a copper mesh, covered with nitrocellulose, then negatively stained with phosphotungstic acid, dried at room temperature and observed under an electron microscope. The results show that DP7-C micelles are spherical and distributed uniformly. Under the electron microscope, the particle size of DP7-C micelles is about 60nm, and the particle size of DP7-C micelle/siRNA complex is about 100nm (see Fig. 14 for the results).

**Example 13 Detection of transfection efficiency of DP7-C/siRNA complex in vitro**

In 24h before transfection, the C26 mouse colon cancer cells or B16 mouse melanoma cells are laid in a 6-well plate with a density of $5\times 10^4$ cells per well, and 2ml of DMEM medium containing 20% FBS is added to each well. FAM-modified no-sense siRNA (FAM-siRNA) is used as a reporter gene to detect transfection efficiency, while PEI25K and Lipofectamin2000 are used as positive controls. During transfection, the medium is firstly replaced with 1ml of serum-free DMEM medium. Subsequently, the gene transfection complexes mixed at different proportions are added to each well, and each complex contains 1μg of FAM-siRNA. Among them, the ratio of siRNA/DP7-C, siRNA/PEI25K and siRNA/Lipofectamin2000 is 1:5, 1:2 and 1:2 respectively. After the medium is incubated at 37°C for 4h, it is replaced by DMEM medium supplemented with 20% FBS and sequentially incubated. After 24h, the transfection is observed under a microscope and photographed. All cells in the wells including floating and adherent cells are collected, washed twice with precooled PBS, and the total fluorescence intensity of each group is counted by flow cytometer (EPICS Elite ESP, USA).

The transfection results are given in Fig. 15. The detection and observation results show that DP7-C could transfect FAM-modified fluorescent siRNA into Ct26 (Fig. 15c) and B16 (Fig. 15b) cells with transfection efficiencies of $42.4\pm3.5\%$ and $53.3\pm4.0\%$ respectively; the corresponding transfection efficiency of PEI complex is $19.0\pm1.8\%$ and $72.0\pm4.1\%$ respectively; and the corresponding transfection efficiency of Lipofectamin2000 complex is $29.5\pm3.2\%$ and $25.6\pm4.2\%$ respectively, which could be directly reflected in Fig. 15a. The results show that DP7-C micelles have higher siRNA transfection efficiency than PEI25K and Lipofectamin2000.

**Example 14 DP7-C cytotoxicity detection**

Cytotoxicity of cations is one of the important factors that restrict the application of siRNA transfection. The toxicity of DP7-C micelles to 293T human embryonic kidney cell line, a normal cell, is detected through cell survival experiments. 293T cells are laid in a 96-well plate at a density of $3\times 10^3$ cells per well, and 100μL of DMEM/20% FBS medium is added to each well and incubated for 24h. In the experiment, three groups are set up, i.e. DP7-C, PEI25K and Lipofectamin2000; and each group had 10 concentration gradients of 0, 12.5, 18.75, 25, 37, 50, 75, 100, 150 and 200 μg/mL respectively. The survival of the cells was detected by the CCK-8 method. After 24h of dosing, 10μL of CCK-8 solution is added to each well and incubated at 37°C for 2h. The absorption value of each well is detected at the wavelength of 630 nm and 450 nm by a microplate reader. The standard curve is drawn and $IC_{50}$ is calculated, and the average value of 6 groups of parallel experiments is taken as the final result.

The detection results are given in Fig. 16. PEI25K and Lipofectamin2000 are highly toxic with $IC_{50}$ less than

20 μg/mL; while DP7-C micelle has small toxicity with $IC_{50}$ exceeding 200 μg/mL and thus it is safe.

**Example 15 C26 mice model of metastatic colon cancer in the abdominal cavity treated by DP7-C-transmitted VEGF siRNA**

In order to further characterize the application potential of DP7-C micelles in tumor therapy, we established a C26 mouse colon cancer peritoneal metastasis model and used DP7-C to transmit anti-VEGF siRNA therapeutic genes for therapeutic research.

(1) Establishment of abdominal metastasis model: on day 0, female BALB/c mice aged 6 - 8 weeks are intraperitoneally injected with 0.1ml of cell suspension (containing about $1\times 10^5$ C26 cells).
(2) On day 3, mice are randomly divided into 4 groups, 8 mice for each group, and are labeled.
(3) Four groups of mice are intraperitoneally injected with 10 doses of normal saline (blank control group), blank DP7-C micelle (17.5μg), DP7-C/no-sense control siRNA (Scramble siRNA) complex (17.5μg/3.5μg) or DP7-C/VEGF siRNA complex (17.5μg/3.5μg) respectively.
(4) On day 20, when the mice in the control group are already very weak, all the mice are killed by cervical dislocation; the tumor tissues in the abdominal cavity as well as the heart, liver, spleen, lung and kidney tissues are immediately collected, weighed and analyzed. The ascites of each group of mice is also collected and measured, and CD31 immunohistochemical staining is performed on tumor tissues of each experimental group. The collected heart, liver, spleen, lung and kidney tissues are analyzed by HE staining.

Fig. 17 shows the therapeutic effect of DP7-C/VEGF siRNA complex which is intraperitoneally injected to treat abdominal metastasis C26 tumor model. Fig. 17a is a photograph of abdominal cavity of a representative mouse in each group of animals. Among them, the average tumor weight is 1.07±0.5g for the DP7-C/VEGF siRNA complex treatment group, 8.82±0.63g for the normal saline control group, 7.94±0.53g for the no-load DP7-C group, and 5.3±0.72g for the DP7-C/no-sense control siRNA complex group (Fig. 17c). The number of metastatic tumor nodule in abdominal cavity is significantly smaller in the mice treated with DP7-C/VEGF siRNA complex, compared with other treatment groups. Therefore, tumor growth in mice treated with DP7-C/VEGF siRNA complex is greatly inhibited.

Meanwhile, as shown in Fig. 17b, the volume of ascites produced by mice in each group is significantly different. The average tumor weight is 0.2±0.1mL for the DP7-C/VEGF siRNA complex treatment group, 1.17±0.4mL for the normal saline control group, 1.17±0.51mL for the blank DP7-C micelle group, and 0.58±0.11mL for the DP7-C/no-sense control siRNA complex group. For mice in each group that are not treated with DP7-C/VEGF siRNA complex, the ascites shows obvious blood red, confirming that mice in these groups have serious mesenteric injury. Compared with other experimental groups, DP7-C/VEGF siRNA complex effectively inhibits the growth of C26 peritoneal metastasis tumor model and the production of accompanying serious tumor infiltration and inflammation.

In addition, CD31 immunohistochemical staining results show that DP7-C/VEGF siRNA complex treatment group has fewer new blood vessels in tumor tissue than other three experimental groups, confirming that DP7-C could effectively inhibit tumor tissue angiogenesis by transmitting anti-VEGF siRNA (Fig. 17d). Meanwhile, HE staining results show (Fig. 17e) that no significant pathological changes are observed in major organ tissues after DP7-C micelles have been intraperitoneally injected, confirming that DP7-C has few side effects.

**Example 16 Subcutaneously implanted tumor model of C26 mice colon cancer treated by DP7-C-transmitted VEGF siRNA**

In order to further characterize the application potential of DP7-C micelles in tumor therapy, we established a subcutaneously implanted tumor model of C26 mouse colon cancer and used DP7-C to transmit anti-VEGF siRNA therapeutic genes for therapeutic research.

(1) On day 0, female BALB/c mice aged 6-8 weeks are subcutaneously inoculated with 0.1mL of cell suspension (containing about $1.5\times 10^6$ C26 cells).
(2) On day 8, when the tumor is palpable, mice are randomly divided into 4 groups, 8 mice for each group, and are labeled.
(3) Four groups of mice are intratumorally injected with 10 doses of normal saline (blank control group), blank DP7-C micelle (25μg), DP7-C/no-sense control siRNA complex (25μg/5μg) or DP7-C/VEGF siRNA complex (25μg/5μg) respectively.
(4) On day 20, when the mice in the control group are already very weak, all the mice are killed by cervical dislocation; the tumor tissues in the abdominal cavity as well as the heart, liver, spleen, lung and kidney tissues are immediately collected, weighed and analyzed. The ascites of each group of mice is also collected and measured, and CD31

immunohistochemical staining is performed on tumor tissues of each experimental group. The collected heart, liver, spleen, lung and kidney tissues are analyzed by HE staining.

Fig. 18 shows the therapeutic effect of DP7-C/VEGF siRNA complex which is intratumorally injected to treat C26 subcutaneously implanted tumor model. Fig. 18a shows a photograph of mouse subcutaneous tumors in each group of animals. Among them, the average tumor weight is 261.4±115.51$mm^3$ for the DP7-C/VEGF siRNA complex treatment group, 577.21±107.46$mm^3$ for the normal saline control group, 357.64±30.56$mm^3$ for the blank DP7-C micelle group, and 474.43±120.67$mm^3$ for the DP7-C/no-sense control siRNA complex group. The volume of subcutaneous tumor of mice treated with DP7-C/VEGF siRNA complex is significantly smaller than that of other treatment groups (Fig. 18b). Therefore, tumor growth in mice treated with DP7-C/VEGF siRNA complex is greatly inhibited.

In addition, CD31 immunohistochemical staining results (Fig. 18c) show that DP7-C/VEGF siRNA complex treatment group has fewer new blood vessels in tumor tissue than other three experimental groups, confirming that DP7-C could effectively inhibit tumor tissue angiogenesis by transmitting anti-VEGF siRNA. Meanwhile, HE staining results (Fig. 18d) show that no significant pathological changes are observed in major organ tissues after DP7-C micelles have been intraperitoneally injected, confirming that DP7-C has few side effects.

**Example 17 B16 mouse model of metastatic melanoma in the lung treated by DP7-C-transmitted VEGF siRNA**

In order to further characterize the application potential of DP7-C micelles in tumor therapy, we established a B16 mouse model of metastatic melanoma in the lung and used DP7-C to transmit anti-VEGF siRNA therapeutic genes for therapeutic research.

(1) On day 0, female C57 mice aged 6-8 weeks are subcutaneously inoculated with 0.1mL of cell suspension (containing about 2.5× $10^5$ B16 cells).
(2) On day 3, mice are randomly divided into 4 groups, 8 mice for each group, and are labeled.
(3) Mice are injected with 7 doses of normal saline (blank control group), blank DP7-C micelle (60μg), DP7-C/no-sense control siRNA complex (60μg/12μg) or DP7-C/VEGF siRNA complex (60μg/12μg) respectively through tail veins.
(4) On day 20, when the mice in the control group are already very weak, all the mice are killed by cervical dislocation; the tumor tissues in the lung as well as the heart, liver, spleen, lung and kidney tissues are immediately collected, weighed and analyzed. The collected heart, liver, spleen, lung and kidney tissues are analyzed by HE staining.

Fig. 19 shows the therapeutic effect of DP7-C/VEGF siRNA complex which is intravenously injected to treat B16 mouse model of metastatic melanoma in the lung. Fig. 19a shows a photograph of lung tumors in mouse in each group of animals. Among them, the average number of tumor nodes is 30±12 for the DP7-C/VEGF siRNA complex treatment group, 172±16 for the normal saline control group, 132±22 for the blank DP7-C micelle group, and 106±15 for the DP7-C/no-sense control siRNA complex group. In addition, the average weight of lung tissue is 0.22±0.02g for the DP7-C/VEGF siRNA complex treatment group, 0.52±0.18g for the normal saline control group, 0.41±0.1g for the blank DP7-C micelle group, and 0.42±0.17g for the DP7-C/no-sense control siRNA complex group. The number of metastatic tumor lung nodules in the lung of mice treated with DP7-C/VEGF siRNA complex is significantly less than that in other treatment groups (Fig. 19a), and the average lung weight of mice is significantly lighter than that in other treatment groups (Fig. 19b). Therefore, tumor growth in mice treated with DP7-C/VEGF siRNA complex is greatly inhibited.

In addition, HE staining results (Fig. 19c) show that no significant pathological changes are observed in major organ tissues after DP7-C micelles have been intraperitoneally injected, confirming that DP7-C has few side effects.

## Claims

1. A hydrophobically modified antimicrobial peptide, **characterized in that** the hydrophobic modification is to couple a hydrophobic fragment at the nitrogen terminal of the antimicrobial peptide, and the amino acid sequence of the antimicrobial peptide is VQWRIRVAVIRK,
**characterized in that**,

    the hydrophobic fragment is succinylated cholesterol, and
    the nitrogen terminal of the antimicrobial peptide is coupled to the hydrophobic segment by the amidation reaction of -CO-OH on the hydrophobic segment with $-NH_2$ on the antimicrobial peptide.

2. The hydrophobically modified antimicrobial peptide according to claim 1, **characterized in that** the antimicrobial

peptide VQRWRIRVAVRK is modified by C-terminal amidation to produce VQWRIRVAVIRK-$NH_2$.

**3.** The hydrophobically modified antimicrobial peptide according to any one of claims 1 to 2, **characterized in that** the structure of the hydrophobically modified antimicrobial peptide is:

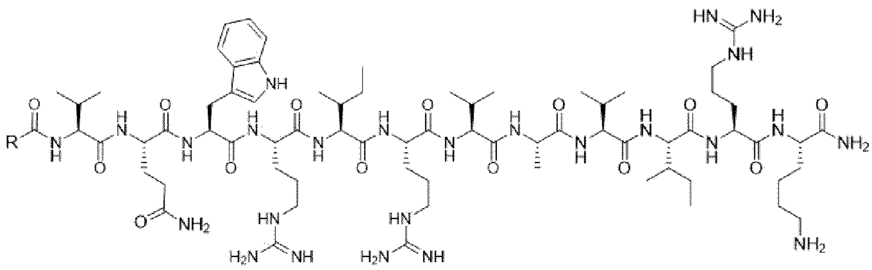

wherein, R is

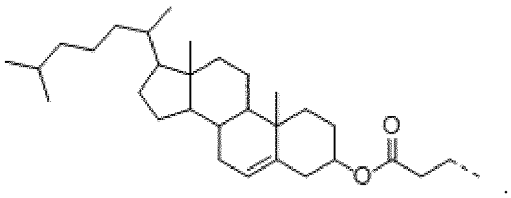

**4.** A micelle prepared from the hydrophobically modified antimicrobial peptide according to any one of claims 1 to 3; preferably the micelle is self-assembled from the hydrophobically modified antimicrobial peptide in solution.

**5.** The micelle according to claim 4, **characterized in that** the micelle is further loaded with at least one of nucleic acid, small molecule drug or protein.

**6.** An antimicrobial drug, **characterized in that** the antimicrobial drug comprises the hydrophobically modified antimicrobial peptide according to any one of claims 1 to 3 or the micelle according to any one of claims 4 to 5; preferably the antimicrobial is antibacterial or antifungal; more preferably the bacteria are at least one of Staphylococcus aureus, Escherichia colibacillus, Acinetobacter baumanmii, Pseudomonas aeruginosa or Salmonella typhi; more preferably the fungi are at least one of Canidia Albicans or Candida parapsilosis.

**7.** The antimicrobial drug according to claim 6, **characterized in that** the antimicrobial drug further comprises other antimicrobial drugs; preferably the other antimicrobial drugs are antibiotics.

**8.** An immunologic adjuvant, **characterized in that** the immunologic adjuvant comprises the hydrophobically modified antimicrobial peptide according to any one of claims 1 to 3 or the micelle according to any one of claims 4 to 5.

**9.** The immune adjuvant according to claim 8, **characterized by** further comprising a single-stranded oligodeoxyribonucleotide CpG ODNs; preferably the ratio of the hydrophobically modified antimicrobial peptide to CpG ODNs is 1: 0.5 to 1:5.

**10.** A nucleic acid transporter, **characterized in that** the nucleic acid transporter comprises a nucleic acid loaded in the hydrophobically modified antimicrobial peptide according to any one of claims 1 to 3 or the micelle according to any one of claims 4 to 5; preferably the nucleic acid is RNA; more preferably the nucleic acid is messenger RNA, siRNA or sgRNA.

**11.** The nucleic acid transporter according to claim 10, **characterized in that** the mass ratio of the hydrophobically modified antimicrobial peptide to the nucleic acid is 1: 1 to 20: 1.

**12.** A method for preparing the nucleic acid transporter according to any one of claims 10 to 11, **characterized by** comprising the following steps:

a weighing a proper amount of the hydrophobically modified antimicrobial peptide according to any one of claims 1 to 3, adding a solution to dissolve, and spontaneously forming micelles; or taking the micelle according to any one of claims 4 to 5 to form a solution;

b adding the nucleic acid into the micellar solution, and incubating at room temperature.

**Patentansprüche**

1. Hydrophob modifiziertes antimikrobielles Peptid, **dadurch gekennzeichnet, dass** die hydrophobe Modifikation ein hydrophobes Fragment an den Stickstoffterminus des antimikrobiellen Peptids koppelt und die Aminosäuresequenz des antimikrobiellen Peptids VQWRIRVAVIRK ist,
**dadurch gekennzeichnet, dass**,

   das hydrophobe Fragment succinyliertes Cholesterin ist und
   der Stickstoffterminus des antimikrobiellen Peptids mittels Amidierungsreaktion von - CO-OH an dem hydrophoben Segment mit -$NH_2$ an dem antimikrobiellen Peptid an das hydrophobe Segment gekoppelt ist.

2. Hydrophob modifiziertes antimikrobielles Peptid nach Anspruch 1, **dadurch gekennzeichnet, dass** das antimikrobielle Peptid VQRWRIRVAVRK mittels C-terminaler Amidierung unter Erhalt von VQWRIRVAVIRK-$NH_2$ modifiziert ist.

3. Hydrophob modifiziertes antimikrobielles Peptid nach einem der Ansprüche 1 bis 2, **dadurch gekennzeichnet, dass** die Struktur des hydrophob modifizierten antimikrobiellen Peptids wie folgt ist:

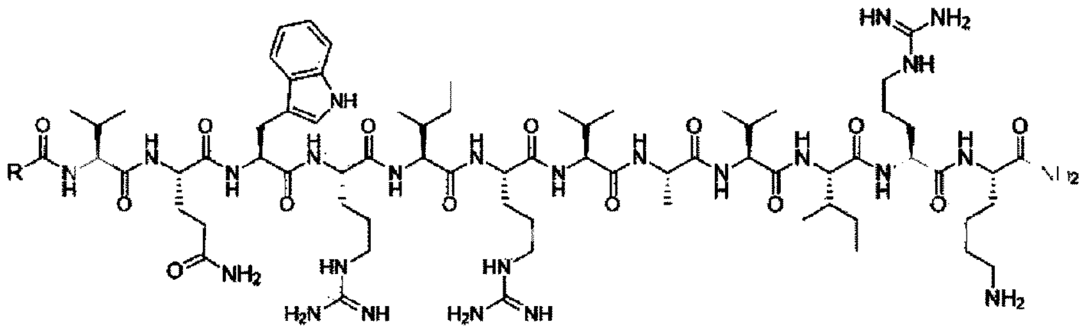

wobei R wie folgt ist:

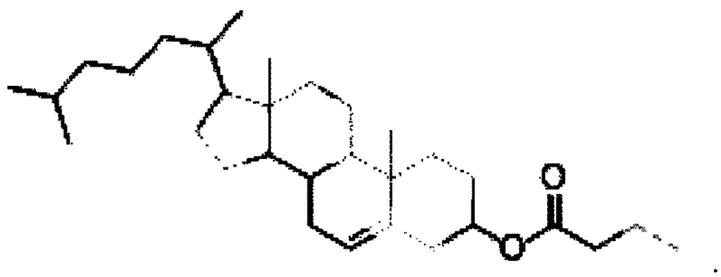

4. Mizelle, hergestellt aus dem hydrophob modifizierten antimikrobiellen Peptid nach einem der Ansprüche 1 bis 3; wobei die Mizelle vorzugsweise aus dem hydrophob modifizierten antimikrobiellen Peptid in Lösung selbstassembliert ist.

5. Mizelle nach Anspruch 4, **dadurch gekennzeichnet, dass** die Mizelle ferner mit wenigstens einem von Nukleinsäure, niedermolekularer Verbindung oder Protein beladen ist.

6. Antimikrobielles Arzneimittel, **dadurch gekennzeichnet, dass** das antimikrobielle Arzneimittel das hydrophob modifizierte antimikrobielle Peptid nach einem der Ansprüche 1 bis 3 oder die Mizelle nach einem der Ansprüche 4 bis 5 umfasst; wobei das antimikrobielle Mittel vorzugsweise antibakteriell oder antimykotisch ist; wobei die Bakterien

besonders bevorzugt wenigstens eines von Staphylococcus aureus, Escherichia colibacillus, Acinetobacter baumanmii, Pseudomonas aeruginosa oder Salmonella typhi sind; wobei die Pilze besonders bevorzugt wenigstens eines von Canidia Albicans oder Candida parapsilosis sind.

7. Antimikrobielles Arzneimittel nach Anspruch 6, **dadurch gekennzeichnet, dass** das antimikrobielle Arzneimittel ferner andere antimikrobielle Arzneimittel umfasst; wobei die anderen antimikrobiellen Arzneimittel vorzugsweise Antibiotika sind.

8. Immunologisches Adjuvans, **dadurch gekennzeichnet, dass** das immunologische Adjuvans das hydrophob modifizierte antimikrobielle Peptid nach einem der Ansprüche 1 bis 3 oder die Mizelle nach einem der Ansprüche 4 bis 5 umfasst.

9. Immunadjuvans nach Anspruch 8, **dadurch gekennzeichnet, dass** es ferner ein einzelsträngiges Oligodesoxyribonukleotid CpG ODNs umfasst; wobei das Verhältnis von hydrophob modifiziertem antimikrobiellem Peptid zu CpG ODNs vorzugsweise bei 1 : 0,5 bis 1 : 5 liegt.

10. Nukleinsäuretransporter, **dadurch gekennzeichnet, dass** der Nukleinsäuretransporter eine Nukleinsäure umfasst, mit der das hydrophob modifizierte antimikrobielle Peptid nach einem der Ansprüche 1 bis 3 oder die Mizelle nach einem der Ansprüche 4 bis 5 beladen ist; wobei die Nukleinsäure vorzugsweise RNA ist; wobei die Nukleinsäure besonders bevorzugt Messenger-RNA, siRNA oder sgRNA ist.

11. Nukleinsäuretransporter nach Anspruch 10, **dadurch gekennzeichnet, dass** das Massenverhältnis des hydrophob modifizierten antimikrobiellen Peptids zu der Nukleinsäure bei 1 : 1 bis 20 : 1 liegt.

12. Verfahren zum Herstellen des Nukleinsäuretransporters nach einem der Ansprüche 10 bis 11, **dadurch gekennzeichnet, dass** es die folgenden Schritte umfasst:

a Abwiegen einer richtigen Menge an hydrophob modifiziertem antimikrobiellem Peptid nach einem der Ansprüche 1 bis 3, Zugeben einer Lösung zum Auflösen und spontanes Bilden von Mizellen; oder Heranziehen der Mizelle nach einem der Ansprüche 4 bis 5 zur Bildung einer Lösung;
b Zugeben der Nukleinsäure zu der mizellaren Lösung und Inkubieren bei Raumtemperatur.

## Revendications

1. Peptide antimicrobien modifié hydrophobiquement, **caractérisé en ce que** la modification hydrophobe consiste à coupler un fragment hydrophobe à la terminaison azotée du peptide antimicrobien, et la séquence d'acides aminés du peptide antimicrobien est VQWRIRVAVIRK,
**caractérisé en ce que**

le fragment hydrophobe est du cholestérol succinylé, et
la terminaison azotée du peptide antimicrobien est couplée au segment hydrophobe par la réaction d'amidation de -CO-OH sur le segment hydrophobe avec $-NH_2$ sur le peptide antimicrobien.

2. Peptide antimicrobien modifié hydrophobiquement selon la revendication 1, **caractérisé en ce que** le peptide antimicrobien VQRWRIRVAVRK est modifié par amidation C-terminale pour produire $VQWRIRVAVIRK-NH_2$.

3. Peptide antimicrobien modifié hydrophobiquement selon l'une quelconque des revendications 1 à 2, **caractérisé en ce que** la structure du peptide antimicrobien modifié hydrophobiquement est :

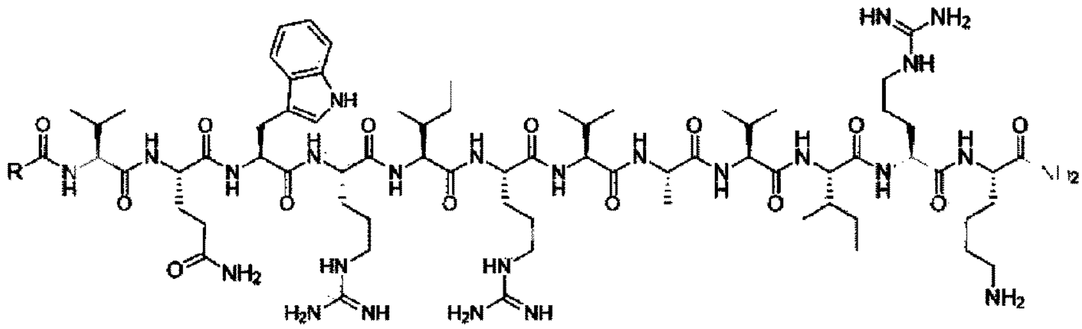

dans laquelle, R est

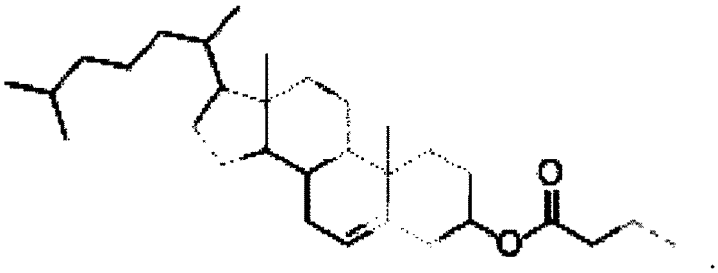

4. Micelle préparée à partir du peptide antimicrobien modifié hydrophobiquement selon l'une quelconque des revendications 1 à 3 ; la micelle étant de préférence auto-assemblée à partir du peptide antimicrobien modifié hydrophobiquement en solution.

5. Micelle selon la revendication 4, **caractérisée en ce que** la micelle est en outre chargée avec au moins un acide nucléique, une petite molécule médicamenteuse ou une protéine.

6. Médicament antimicrobien, **caractérisé en ce que** le médicament antimicrobien comprend le peptide antimicrobien modifié hydrophobiquement selon l'une quelconque des revendications 1 à 3 ou la micelle selon l'une quelconque des revendications 4 à 5 ; l'antimicrobien étant de préférence antibactérien ou antifongique ; les bactéries étant de manière davantage préférée au moins une parmi Staphylococcus aureus, Escherichia colibacillus, Acinetobacter baumanmii, Pseudomonas aeruginosa ou Salmonella typhi ; les champignons étant de manière davantage préférée au moins un parmi Canidia Albicans ou Candida parapsilosis.

7. Médicament antimicrobien selon la revendication 6, **caractérisé en ce que** le médicament antimicrobien comprend en outre d'autres médicaments antimicrobiens ; les autres médicaments antimicrobiens étant de préférence des antibiotiques.

8. Adjuvant immunologique, **caractérisé en ce que** l'adjuvant immunologique comprend le peptide antimicrobien modifié hydrophobiquement selon l'une quelconque des revendications 1 à 3 ou la micelle selon l'une quelconque des revendications 4 à 5.

9. Adjuvant immunitaire selon la revendication 8, **caractérisé en ce qu'**il comprend en outre un oligodésoxyribonucléotide simple brin CpG ODN ; le rapport entre le peptide antimicrobien modifié hydrophobiquement et les CpG ODN étant de préférence de 1:0,5 à 1:5.

10. Transporteur d'acide nucléique, **caractérisé en ce que** le transporteur d'acide nucléique comprend un acide nucléique chargé dans le peptide antimicrobien modifié hydrophobiquement selon l'une quelconque des revendications 1 à 3 ou la micelle selon l'une quelconque des revendications 4 à 5 ; l'acide nucléique étant de préférence un ARN ; l'acide nucléique étant de manière davantage préférée un ARN messager, un pARNi ou un ARNsg.

11. Transporteur d'acide nucléique selon la revendication 10, **caractérisé en ce que** le rapport massique entre le peptide antimicrobien modifié hydrophobiquement et l'acide nucléique est de 1:1 à 20:1.

**12.** Procédé de préparation du transporteur d'acide nucléique selon l'une quelconque des revendications 10 à 11, **caractérisé en ce qu'**il comprend les étapes suivantes :

a la pesée d'une quantité appropriée du peptide antimicrobien modifié hydrophobiquement selon l'une quelconque des revendications 1 à 3, l'ajout d'une solution pour dissoudre, et la formation spontanée de micelles ; ou le prélèvement de la micelle selon l'une quelconque des revendications 4 à 5 pour former une solution ;
b l'ajout de l'acide nucléique dans la solution micellaire, et l'incubation à température ambiante.

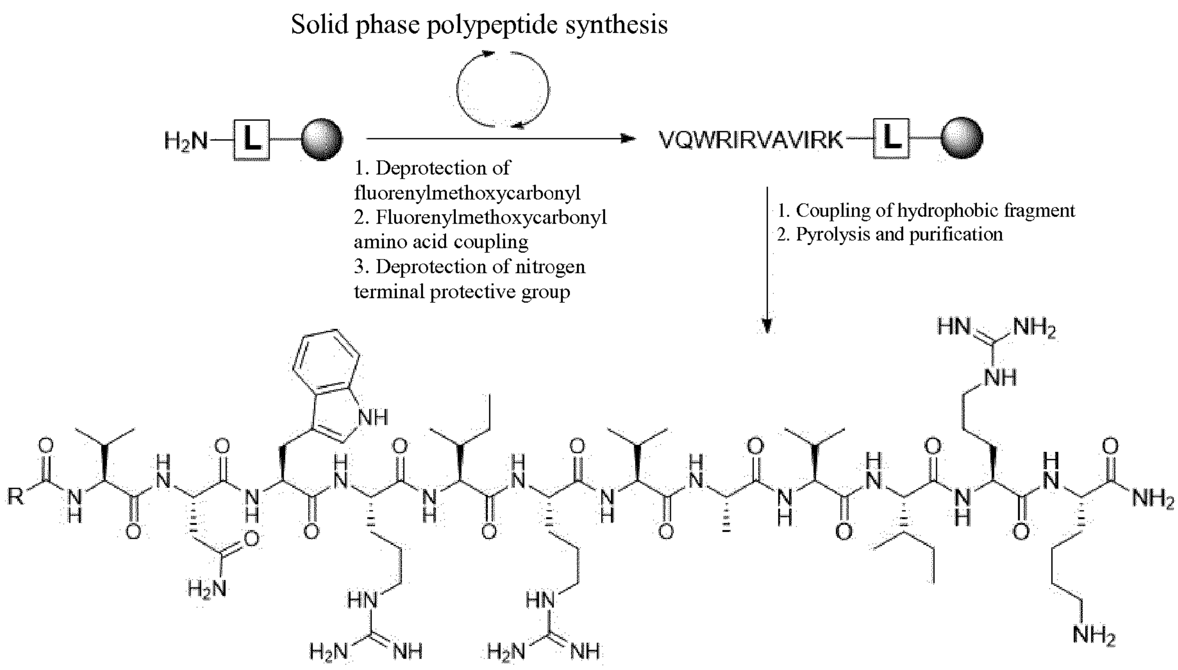
Solid phase polypeptide synthesis
VQWRIRVAVIRK
1. Deprotection of fluorenylmethoxycarbonyl
2. Fluorenylmethoxycarbonyl amino acid coupling
3. Deprotection of nitrogen terminal protective group
1. Coupling of hydrophobic fragment
2. Pyrolysis and purification

R is 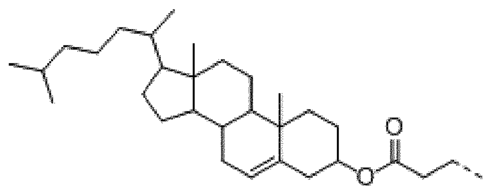 , 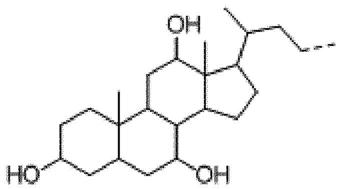 ,

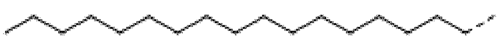 , 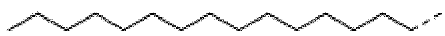 or

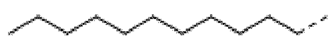 .

**Fig. 1**

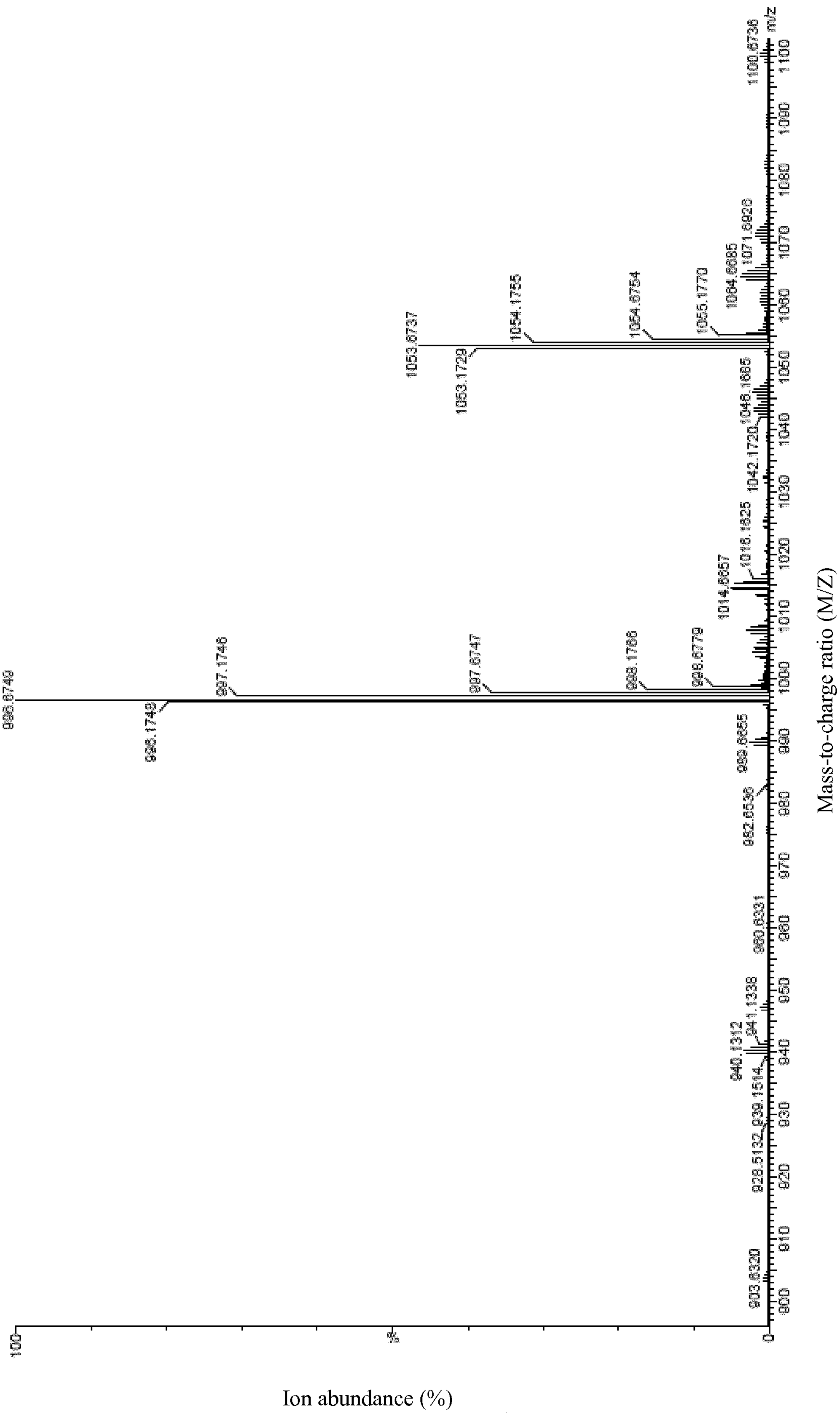
996.6749
996.1748
997.1746
997.6747
998.1766
998.6779
989.6655
982.6536
960.6331
940.1312
941.1338
928.5132
939.1514
903.6320
1014.6657
1018.1625
1042.1720
1046.1685
1053.6737
1053.1729
1054.1755
1054.6754
1055.1770
1064.6685
1071.6926
1100.6736
m/z
100
%
0
Mass-to-charge ratio (M/Z)
Ion abundance (%)

Fig. 2A.

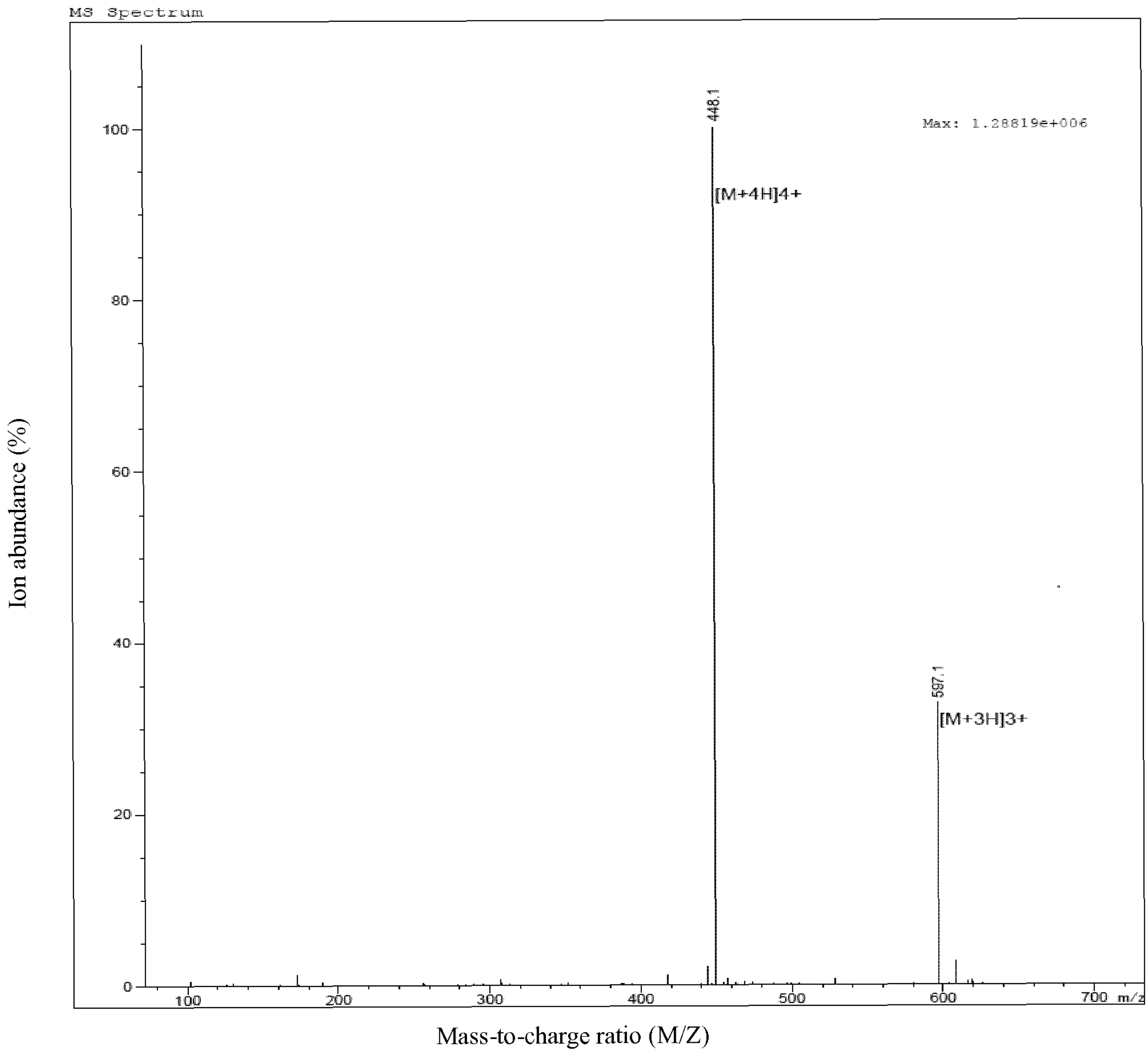
MS Spectrum
Max: 1.28819e+006
448.1
[M+4H]4+
597.1
[M+3H]3+
Ion abundance (%)
0
20
40
60
80
100
100
200
300
400
500
600
700 m/z
Mass-to-charge ratio (M/Z)

Fig. 2B

Fig. 2

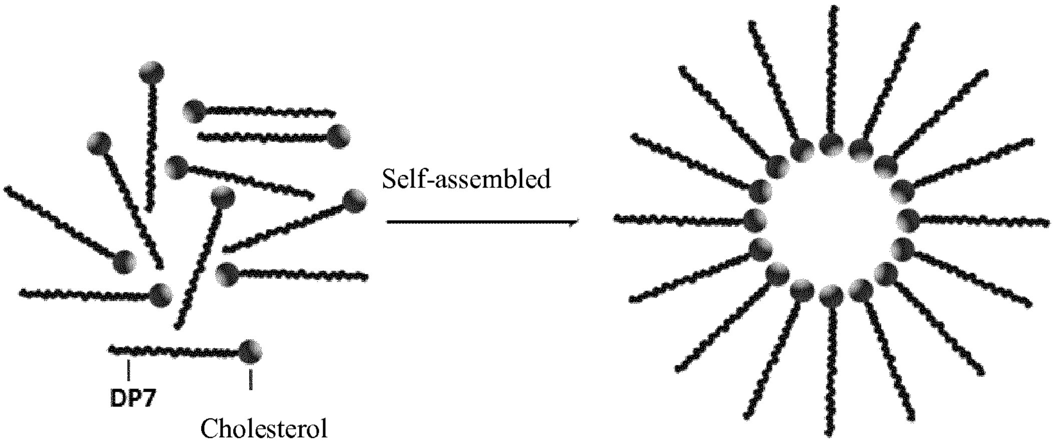


Fig. 3A.

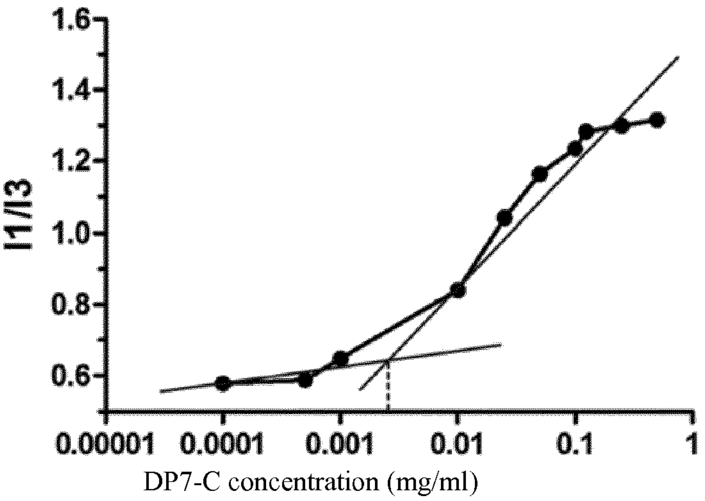


Fig. 3B.

**Fig. 3**

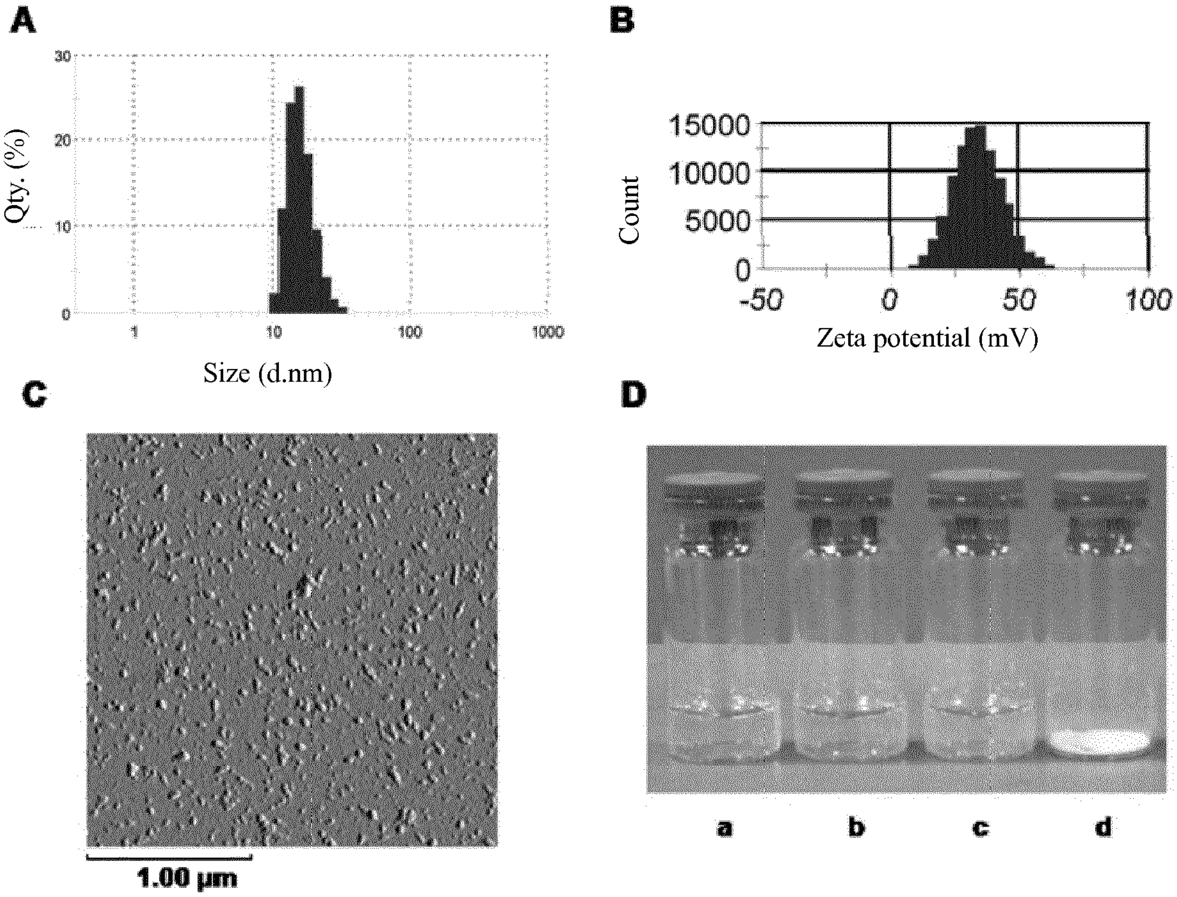


Fig. 4

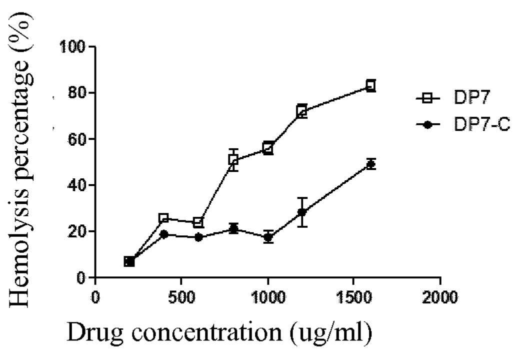


Fig. 5A

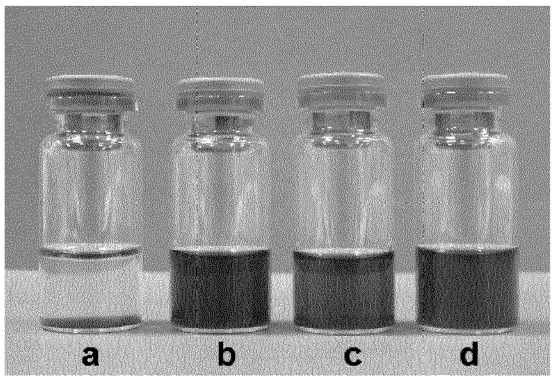


Fig. 5B.

Fig. 5

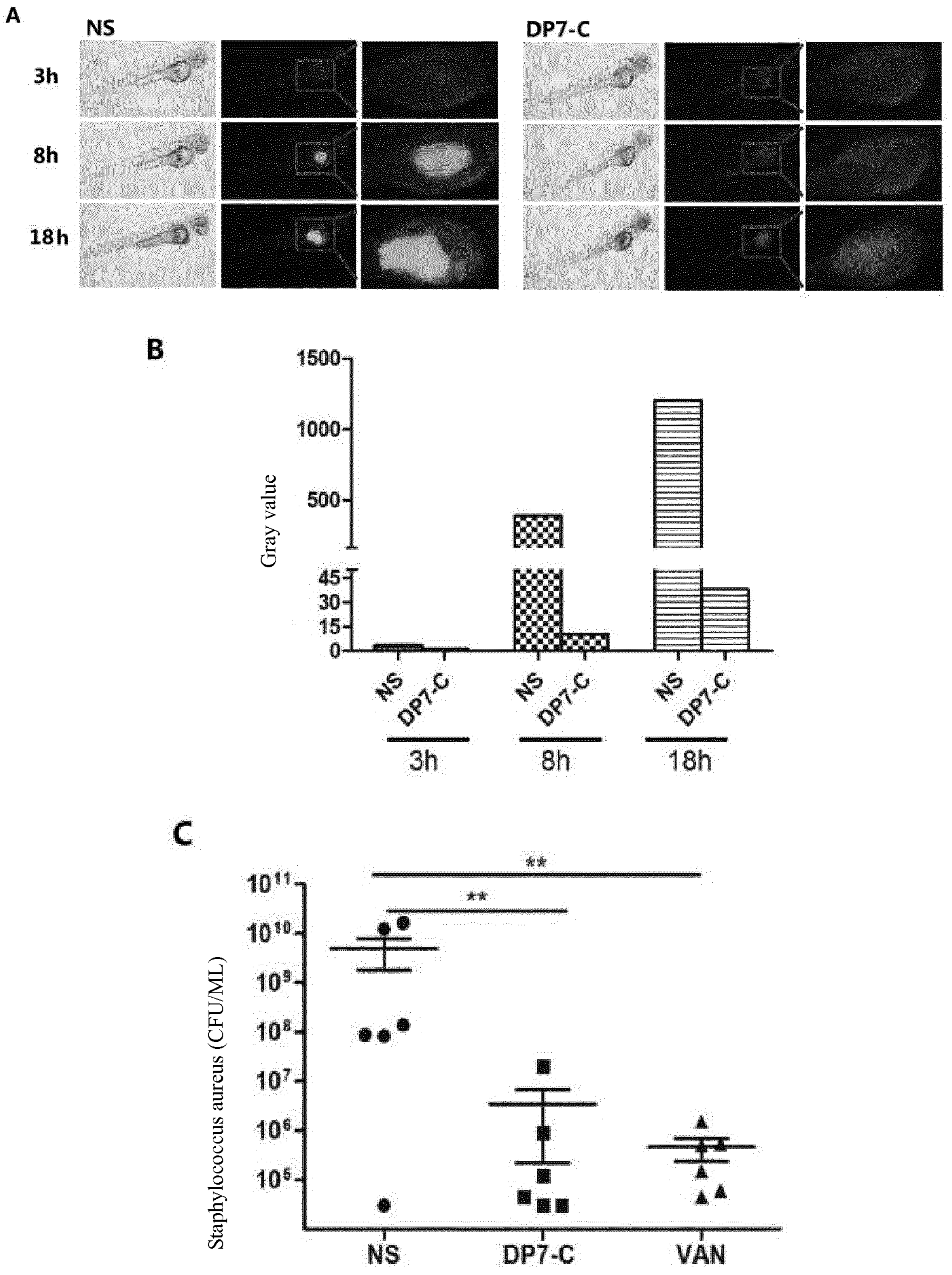
A
NS
DP7-C
3h
8h
18h
B
Gray value
1500
1000
500
45
30
15
0
NS
DP7-C
3h
8h
18h
C
Staphylococcus aureus (CFU/ML)
$10^{11}$
$10^{10}$
$10^9$
$10^8$
$10^7$
$10^6$
$10^5$
**
NS
DP7-C
VAN

Fig. 6

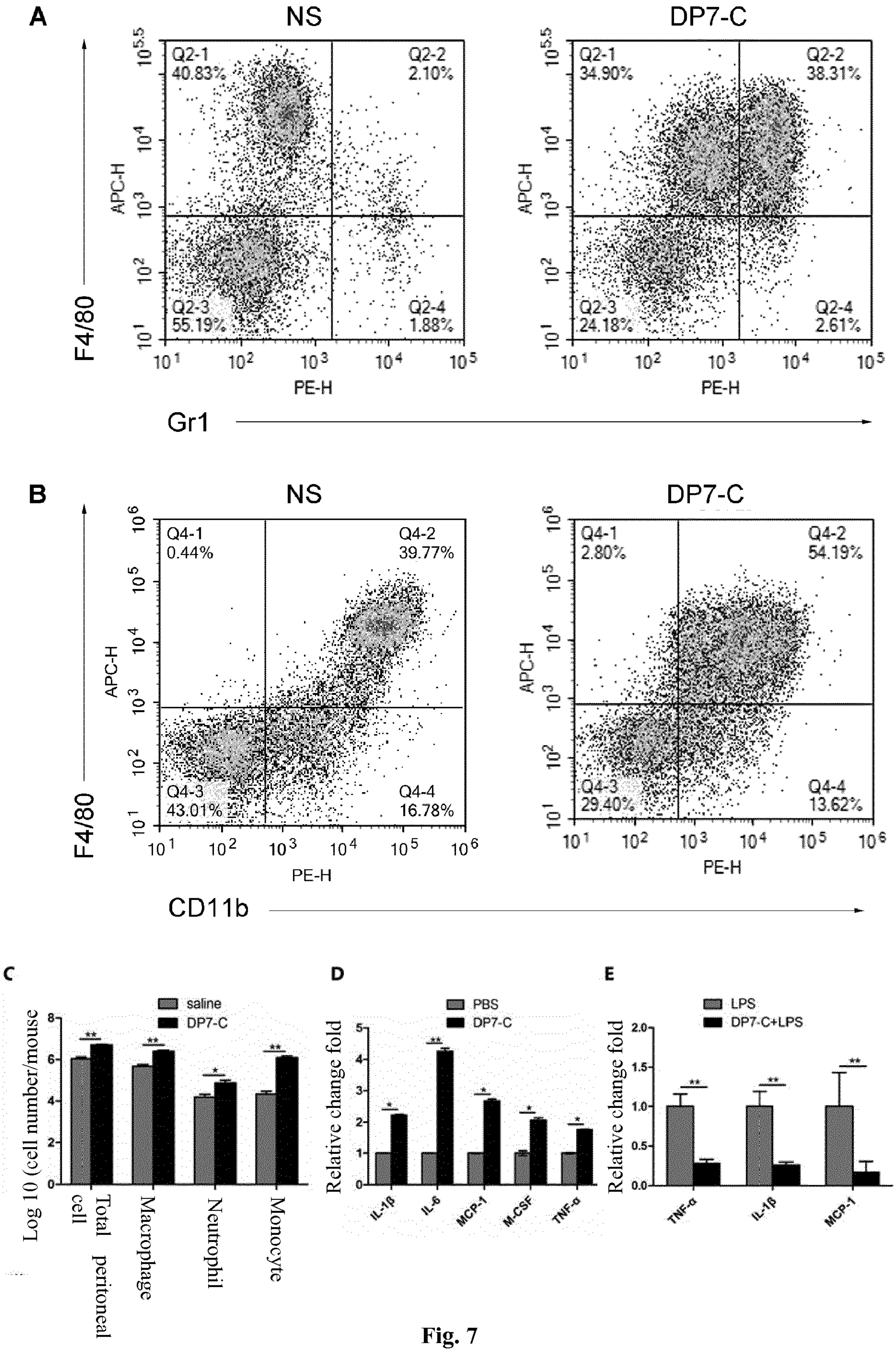
A
NS
DP7-C
Q2-1
40.83%
Q2-2
2.10%
Q2-3
55.19%
Q2-4
1.88%
Q2-1
34.90%
Q2-2
38.31%
Q2-3
24.18%
Q2-4
2.61%
APC-H
PE-H
F4/80
Gr1
B
NS
DP7-C
Q4-1
0.44%
Q4-2
39.77%
Q4-3
43.01%
Q4-4
16.78%
Q4-1
2.80%
Q4-2
54.19%
Q4-3
29.40%
Q4-4
13.62%
APC-H
PE-H
F4/80
CD11b
C
saline
DP7-C
Log 10 (cell number/mouse
Total peritoneal cell
Macrophage
Neutrophil
Monocyte
D
PBS
DP7-C
Relative change fold
IL-1β
IL-6
MCP-1
M-CSF
TNF-α
E
LPS
DP7-C+LPS
Relative change fold
TNF-α
IL-1β
MCP-1

Fig. 7

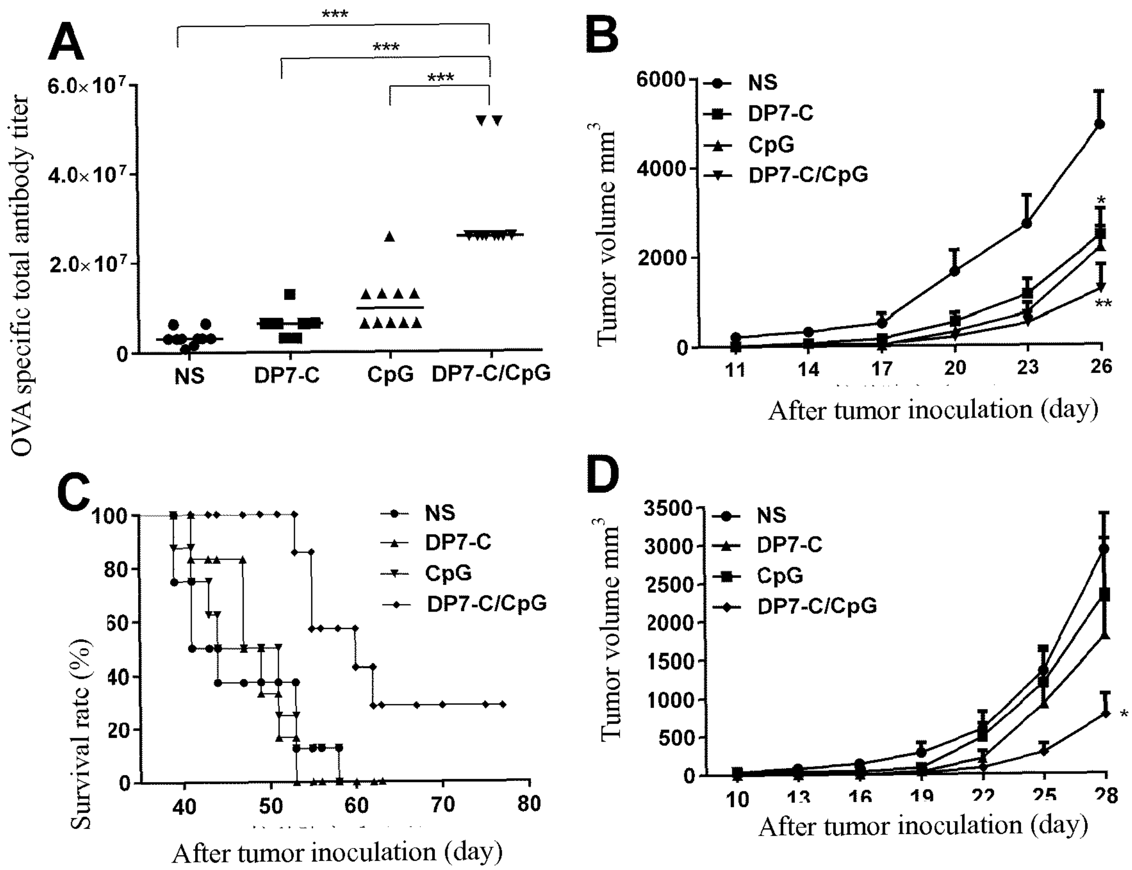
A
***
***
***
OVA specific total antibody titer
6.0×10^7
4.0×10^7
2.0×10^7
0
NS
DP7-C
CpG
DP7-C/CpG
B
Tumor volume mm^3
6000
4000
2000
0
NS
DP7-C
CpG
DP7-C/CpG
*
**
11
14
17
20
23
26
After tumor inoculation (day)
C
Survival ratc (%)
100
80
60
40
20
0
NS
DP7-C
CpG
DP7-C/CpG
40
50
60
70
80
After tumor inoculation (day)
D
Tumor volume mm^3
3500
3000
2500
2000
1500
1000
500
0
NS
DP7-C
CpG
DP7-C/CpG
*
10
28
After tumor inoculation (day)

**Fig. 8**

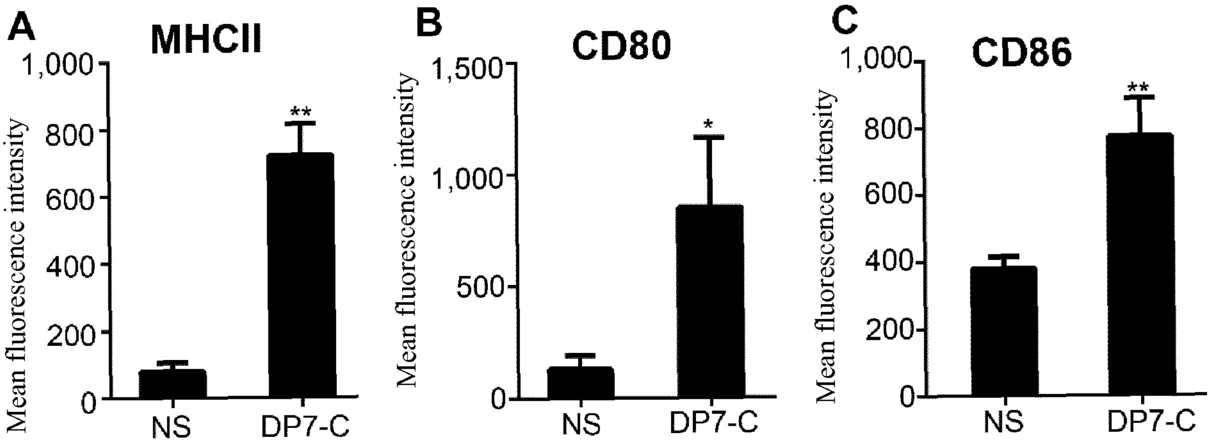
A
MHCII
Mean fluorescence intensity
1,000
800
600
400
200
0
**
NS
DP7-C
B
CD80
Mean fluorescence intensity
1,500
1,000
500
0
*
NS
DP7-C
C
CD86
Mean fluorescence intensity
1,000
800
600
400
200
0
**
NS
DP7-C

Fig. 9

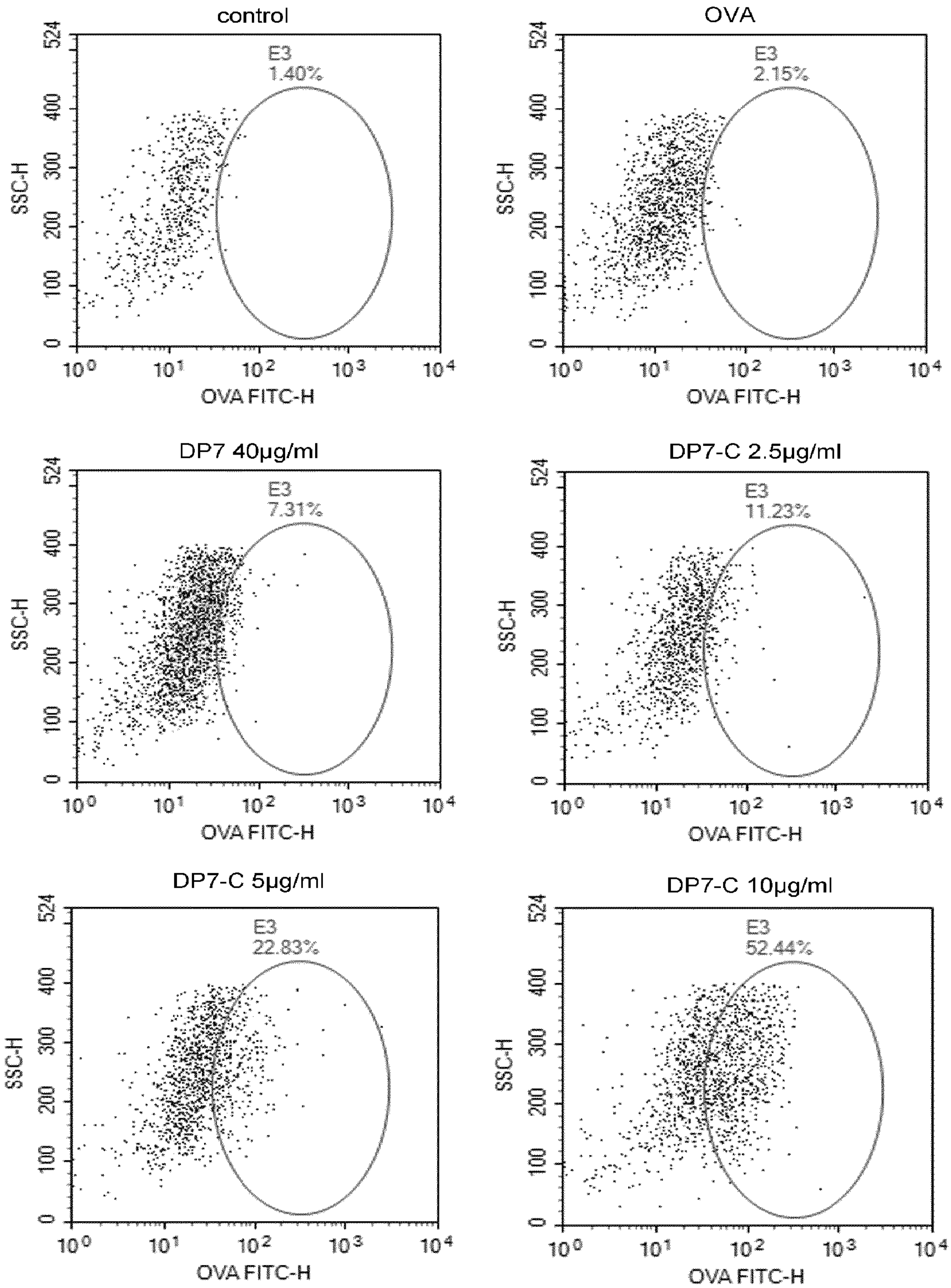
control
OVA
DP7 40μg/ml
DP7-C 2.5μg/ml
DP7-C 5μg/ml
DP7-C 10μg/ml
E3
1.40%
2.15%
7.31%
11.23%
22.83%
52.44%
SSC-H
OVA FITC-H
0
100
200
300
400
524
10⁰
10¹
10²
10³
10⁴

Fig. 10

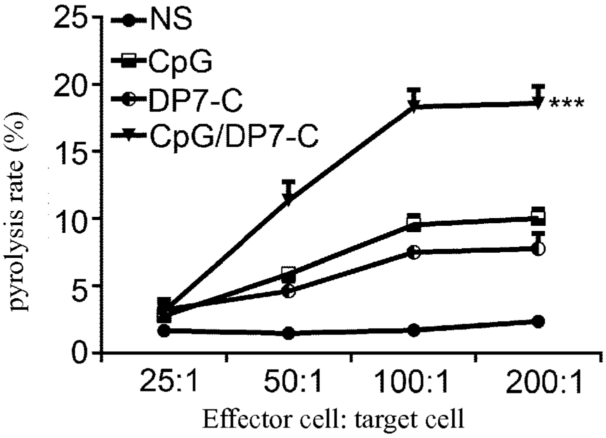
NS
CpG
DP7-C
CpG/DP7-C
***
pyrolysis rate (%)
25
20
15
10
5
0
25:1
50:1
100:1
200:1
Effector cell: target cell

Fig. 11

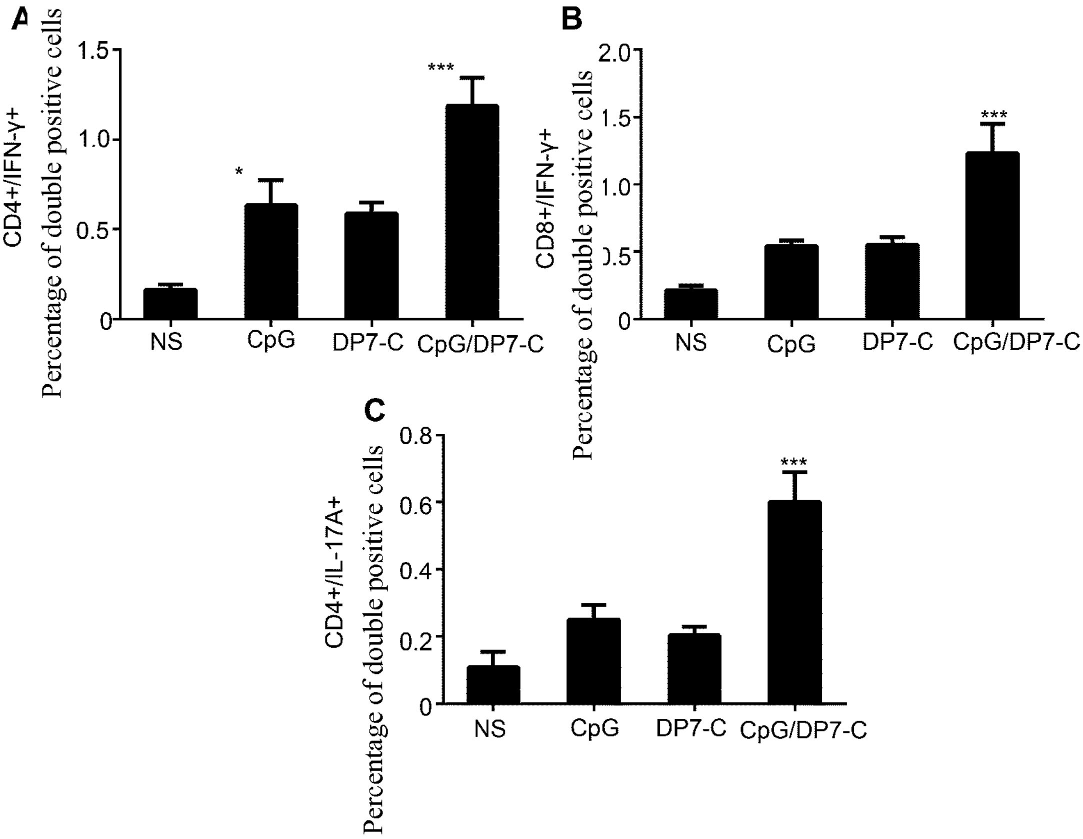
A
CD4+/IFN-γ+
Percentage of double positive cells
1.5
1.0
0.5
0
*
***
NS
CpG
DP7-C
CpG/DP7-C
B
CD8+/IFN-γ+
Percentage of double positive cells
2.0
1.5
1.0
0.5
0
***
NS
CpG
DP7-C
CpG/DP7-C
C
CD4+/IL-17A+
Percentage of double positive cells
0.8
0.6
0.4
0.2
0
***
NS
CpG
DP7-C
CpG/DP7-C

Fig. 12

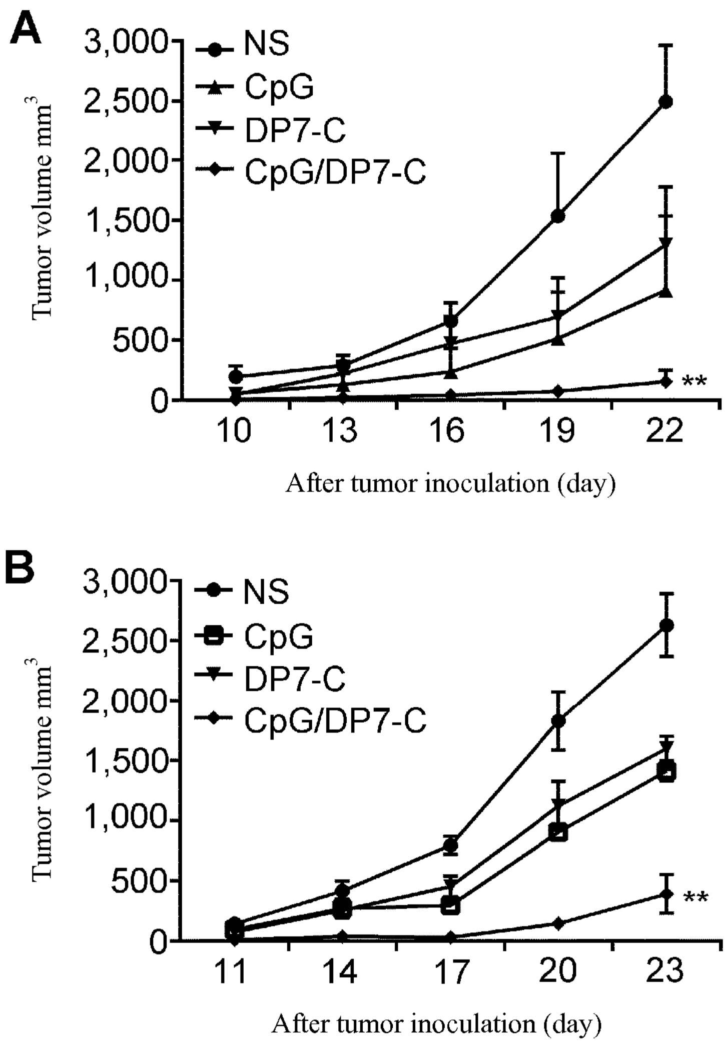
A
3,000
2,500
2,000
1,500
1,000
500
0
Tumor volume mm3
NS
CpG
DP7-C
CpG/DP7-C
**
10
13
16
19
22
After tumor inoculation (day)
B
3,000
2,500
2,000
1,500
1,000
500
0
Tumor volume mm3
NS
CpG
DP7-C
CpG/DP7-C
**
11
14
17
20
23
After tumor inoculation (day)

Fig. 13

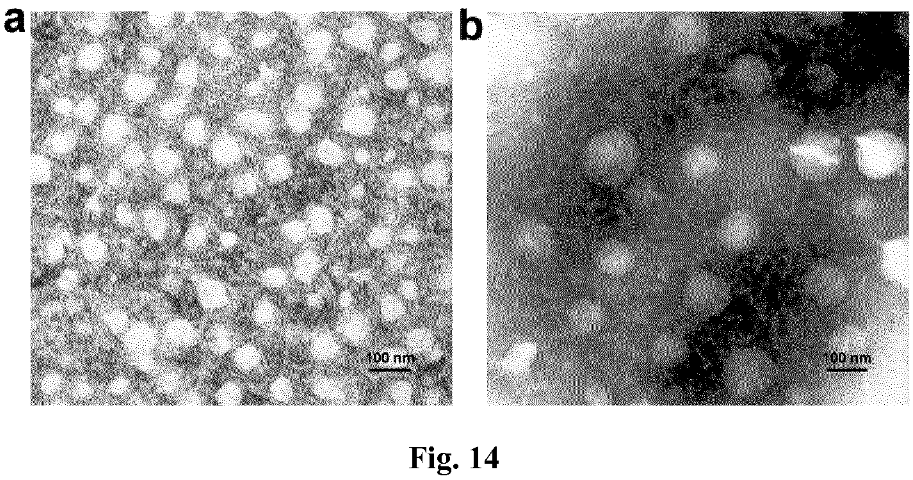
a
b
100 nm
100 nm

**Fig. 14**

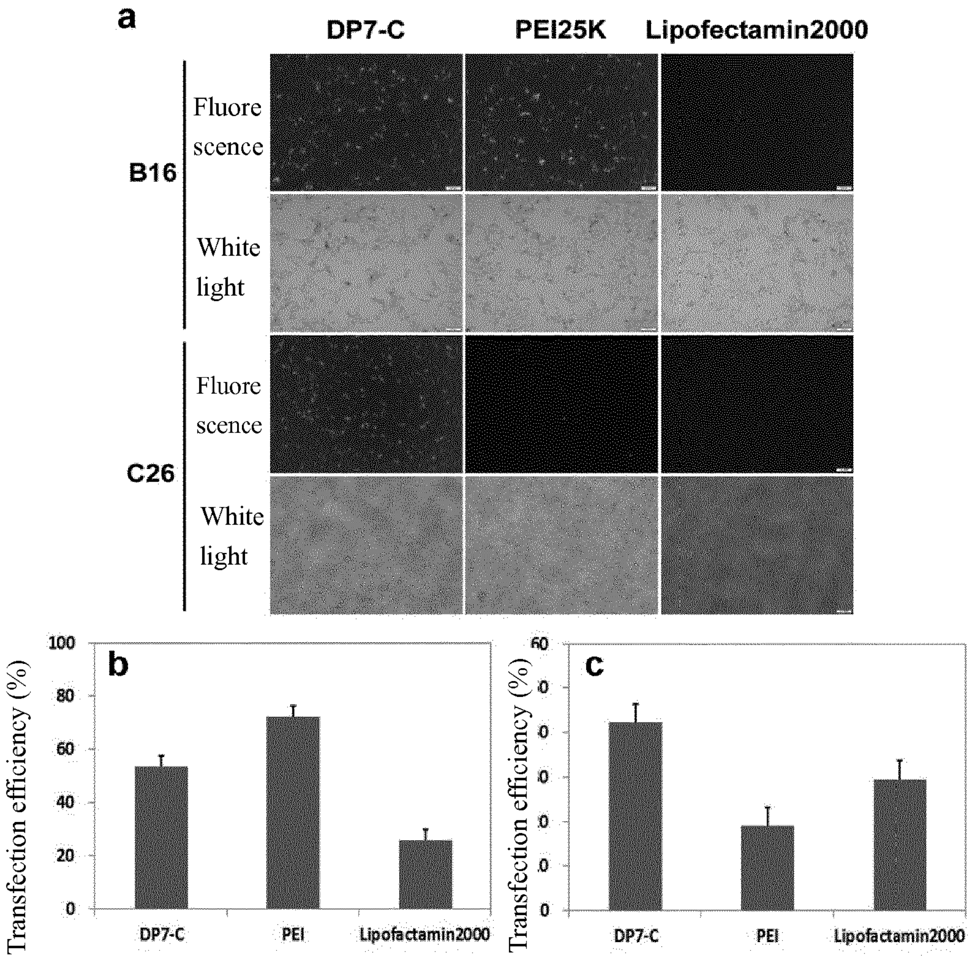
a
DP7-C
PEI25K
Lipofectamin2000
B16
Fluore scence
White light
C26
Fluore scence
White light
b
Transfection efficiency (%)
100
80
60
40
20
0
DP7-C
PEI
Lipofactamin2000
c
Transfection efficiency (%)
60
0
DP7-C
PEI
Lipofactamin2000

Fig. 15

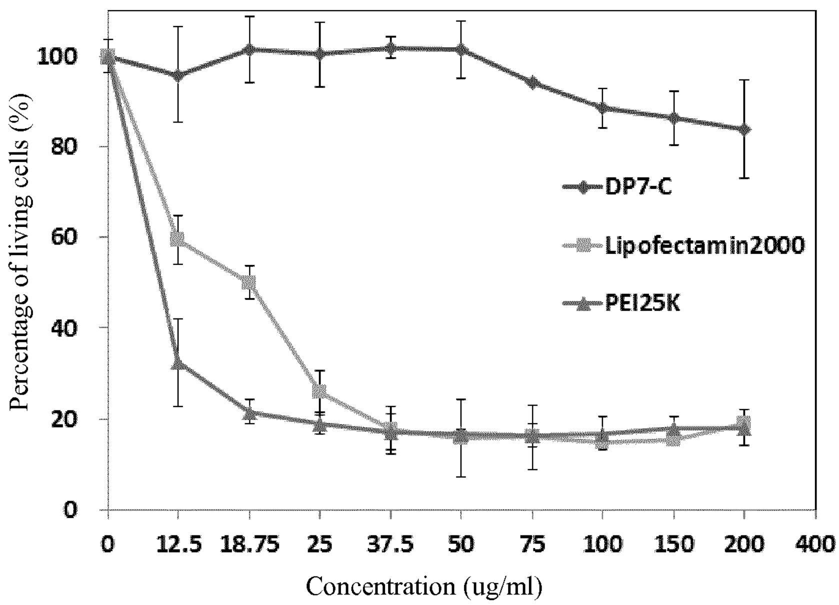
Percentage of living cells (%)
100
80
60
40
20
0
DP7-C
Lipofectamin2000
PEI25K
0
12.5
18.75
25
37.5
50
75
100
150
200
400
Concentration (ug/ml)

Fig. 16

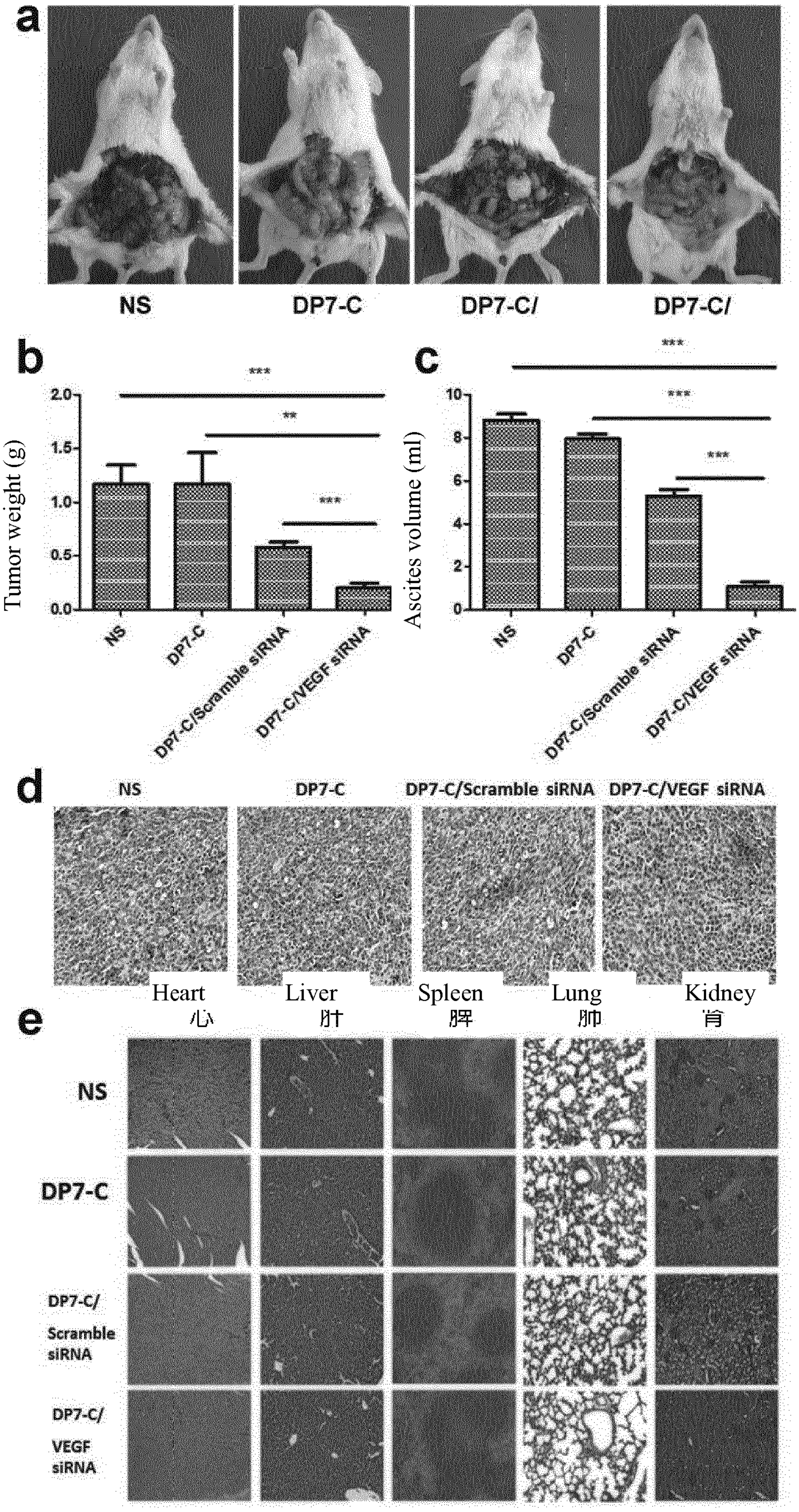

a
NS
DP7-C
DP7-C/
DP7-C/
b
Tumor weight (g)
2.0
1.5
1.0
0.5
0.0
***
**
***
NS
DP7-C
DP7-C/Scramble siRNA
DP7-C/VEGF siRNA
c
Ascites volume (ml)
10
8
6
4
2
0
***
***
***
NS
DP7-C
DP7-C/Scramble siRNA
DP7-C/VEGF siRNA
d
NS
DP7-C
DP7-C/Scramble siRNA
DP7-C/VEGF siRNA
e
Heart
心
Liver
肝
Spleen
脾
Lung
肺
Kidney
肾
NS
DP7-C
DP7-C/
Scramble
siRNA
DP7-C/
VEGF
siRNA


Fig. 17

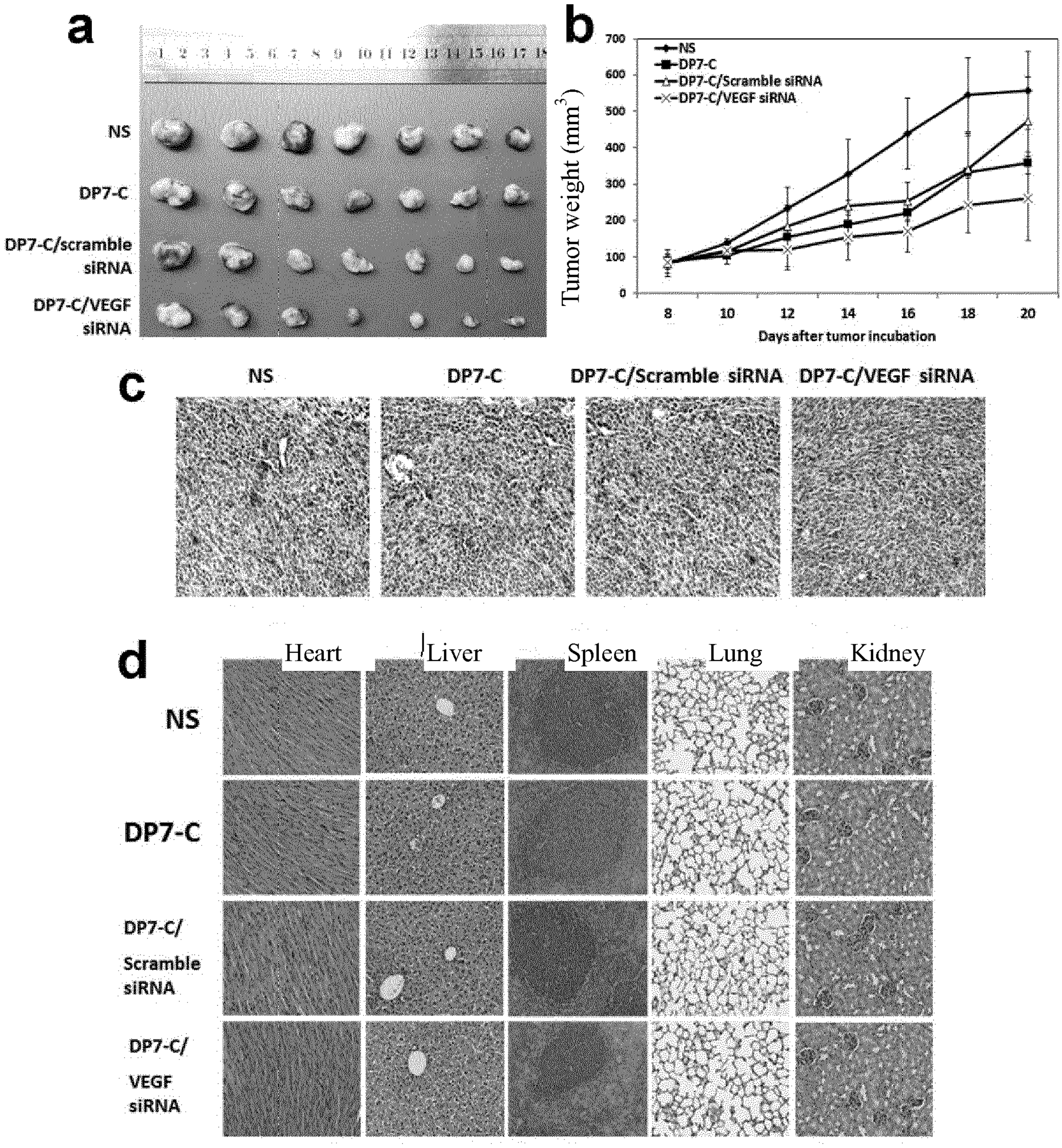
a
NS
DP7-C
DP7-C/scramble siRNA
DP7-C/VEGF siRNA
b
NS
DP7-C
DP7-C/Scramble siRNA
DP7-C/VEGF siRNA
Tumor weight (mm3)
Days after tumor incubation
c
NS
DP7-C
DP7-C/Scramble siRNA
DP7-C/VEGF siRNA
d
Heart
Liver
Spleen
Lung
Kidney
NS
DP7-C
DP7-C/ Scramble siRNA
DP7-C/ VEGF siRNA

Fig. 18

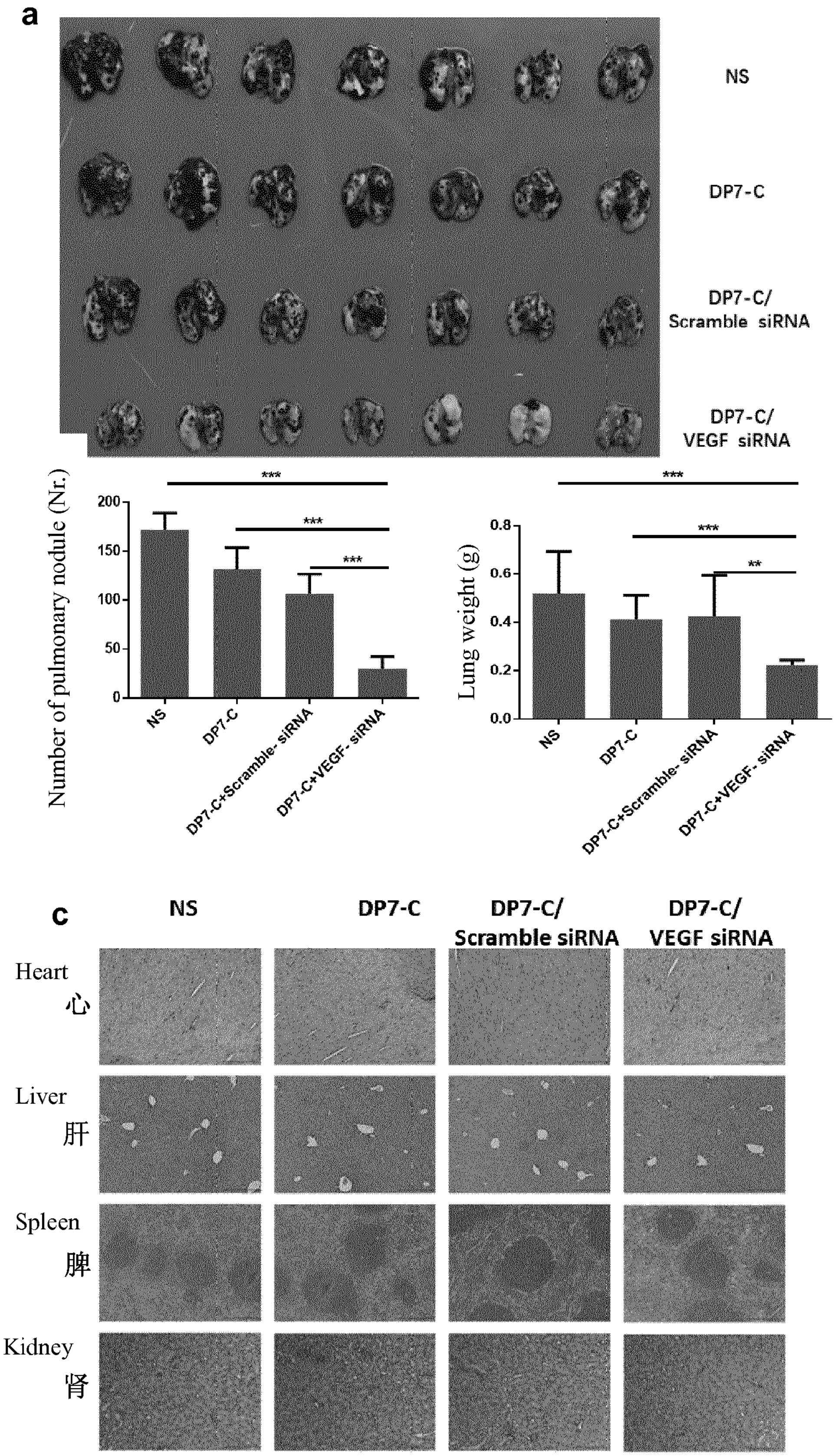
a
NS
DP7-C
DP7-C/
Scramble siRNA
DP7-C/
VEGF siRNA
Number of pulmonary nodule (Nr.)
200
150
100
50
0
***
***
***
NS
DP7-C
DP7-C+Scramble- siRNA
DP7-C+VEGF- siRNA
Lung weight (g)
0.8
0.6
0.4
0.2
0.0
***
***
**
NS
DP7-C
DP7-C+Scramble- siRNA
DP7-C+VEGF- siRNA
c
NS
DP7-C
DP7-C/
Scramble siRNA
DP7-C/
VEGF siRNA
Heart
心
Liver
肝
Spleen
脾
Kidney
肾

Fig. 19

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 08022444 A
- CN 103467579 B

### Non-patent literature cited in the description


- **WU et al.** In vitro and in vivo activities of antimicrobial peptides developed using an amino acid-based activity prediction method. *ANTIMICROBIAL AGENTS AND CHEMOTHERAPY,* 2014, vol. 58, 5342-5349
- **BLUHM et al.** N-terminal Ile-Om- and Trp-Om-motif repeats enhance membrane interaction and increase the antimicrobial activity of Apidaecins against Pseudomonas aeruginosa. *FRONTIERS IN CELL AND DEVELOPMENTAL BIOLOGY,* 2016, vol. 4
- **LIU LIHONG et al.** Self-assembled cationic peptide nanoparticles as an efficient antimicrobial agent. *NATURE NANOTECHNOLOGY,* 2009, vol. 4, 457-463
- **TIAN XIBO et al.** Role of peptide self-assembly in antimicrobial peptides. *JOURNAL OF PEPTIDE SCIENCE,* 2015, vol. 21, 530-539